# EUROPÄISCHE PATENTSCHRIFT

(11) **EP 1 833 830 B1**
(45) Veröffentlichungstag und Bekanntmachung des Hinweises auf die Patenterteilung: **14.10.2009**
(21) Anmeldenummer: 05850343.4
(22) Anmeldetag: 19.12.2005
(51) Int. Cl.: C07D 471/04, A61K 31/435, A61P 35/00

(54) **CHINOLINDERIVAT, DESSEN VERWENDUNG, HERSTELLUNG UND DIESES ENTHALTENDES ARZNEIMITTEL**
QUINOLINE DERIVATIVE, USE AND PRODUCTION THEREOF, AND DRUG CONTAINING THE SAME
DERIVE DE QUINOLEINE, SON UTILISATION, SA FABRICATION ET MEDICAMENT LE CONTENANT

(30) Priorität: 22.12.2004 DE 102004063223; 07.01.2005 US 641733 P
(43) Veröffentlichungstag der Anmeldung: 19.09.2007
(73) Patentinhaber: Bayer Schering Pharma Aktiengesellschaft, 13353 Berlin (DE)
(72) Erfinder: SCHWEDE, Wolfgang, 16548 Glienicke (DE); JAROCH, Stefan, 10629 Berlin (DE); BADER, Benjamin, 13467 Berlin (DE); HILLIG, Roman, 10825 Berlin (DE); TER LAAK, Antonius, 12159 Berlin (DE); ZOPF, Dieter, 14167 Berlin (DE)
(86) Internationale Anmeldenummer: PCT/EP2005/013955
(87) Internationale Veröffentlichungsnummer: WO 2006/066955

(56) Entgegenhaltungen:
- EP-A- 0 187 705
- WO-A-20/04006846

## Beschreibung

Die Erfindung betrifft bestimmte Chinolinderivate, deren Herstellung und Verwendung als Inhibitor von Proteinkinasen, insbesondere von Eph (*e*rythropoetin-*p*roducing *h*epatoma amplified sequence) - Rezeptoren zur Behandlung verschiedener Erkrankungen.

Proteintyrosinkinasen katalysieren die Phosphorylierung spezifischer Tyrosinreste in verschiedenen Proteinen. Solche Phosphorylierungsreaktionen spielen bei einer Vielzahl von zellulären Prozessen eine Rolle, die in der Regulation des Wachstums und der Differenzierung von Zellen involviert sind. Proteintyrosinkinasen werden in Rezeptor- und Nicht-Rezeptortyrosinkinasen eingeteilt. Die Familie der Rezeptortyrosinkinasen (RTKs) besteht aus 58 Kinasen (Manning G. et al. 2002, Science 298, 1912-1934). RTKs besitzen eine extrazelluläre Ligandenbindungsdomäne, eine Transmembrandomäne und eine intrazelluläre Domäne, die in der Regel die Tyrosinkinaseaktivität enthält. RTKs vermitteln die Signalweiterleitung von extrazellulären Stimulatoren wie z.B. Wachstumsfaktoren. Die Ligandenbindung führt zur Dimerisierung der RTKs und der wechselseitigen Autophosphorylierung ihrer intrazellulären Domänen. In Abhängigkeit vom Zelltyp werden dadurch spezifische intrazelluläre Bindungsproteine rekrutiert (u.a. Nicht-Rezeptortyrosinkinasen), über die eine Signalverarbeitung in der Zelle erfolgt (Schlessinger J. 2000, Cell 103, 211-225). Zu diesen zählen Rezeptorfamilien von Wachstumsfaktoren wie EGF (epidermal growth factor), VEGF (vascular endothelial growth factor), FGF (fibroblast growth factor), PDGF (platelet derived growth factor) und NGF (nerve growth factor), sowie der Insulinrezeptoren und die große Familie der Ephrinrezeptoren und andere.

Die größte Familie innerhalb der RTKs nehmen die Ephrin (Eph)-Rezeptoren ein. Sie teilen sich entsprechend ihrer sequenziellen Verwandtschaft und ihrer Ligandenspezifität in die Gruppe der EphA-Rezeptoren (9 Mitglieder) und der EphB-Rezeptoren (6 Mitglieder) auf (Kullander K. and Klein R. 2002, Nat.Rev.Mo/.Cel/ Biol. 3, 475-486; Cheng N. et al. 2002, Cyt. and growth factor Rev. 13, 75-85.). Eph-Rezeptoren werden von membranständigen Liganden der EphrinA- bzw. EphrinB-Familie aktiviert. EphrinAs sind über Glykolipide (GPI) in der Zellmembran verankert, während EphrinBs eine Transmembranregion und eine intrazelluläre Domäne besitzen. Die Interaktion zwischen Ephrinen und den Eph-Rezeptoren führt zu einer bidirektionellen Signalübertragung in den ephrinexprimierenden und in den Eph-Rezeptor tragenden Zellen. Ephrine und Eph-Rezeptoren spielen in einer Vielzahl von morphogenetischen Prozessen in der Embryonalentwicklung und im adulten Organismus eine Rolle. Sie sind involviert in der embryonalen Musterbildung, in der Entwicklung des Blutgefäßsystems (Gerety S.S: et al 1999, Mol. Cell 4, 403-414) und in der Etablierung von neuronalen Verschaltungen (Flanagan, J.G. and Vanderhaeghen, P., 1998, Annu.Rev.Neurosci. 21, 306-354). Im adulten Organismus sind sie bei Neovascularisierungsprozesses z.B. bei der Tumorentstehung und bei der Endometriose, sowie bei der Morphogenese des Darmepithels beteiligt (Batlle E. et al. 2002, Cell 111:251-63.). Auf zellulärer Ebene vermitteln sie Migration, Adhäsion und juxtacrine Zellkontakte. Erhöhte Expression von Eph-Rezeptoren, wie z.B. EphB2 und EphB4 wurde auch in verschiedenen Tumorgeweben, wie z.B. Brust- und Darmtumoren beobachtet (Nakamoto M. and Bergemann A.D. 2002, Mic.Res.Tech. 59, 58-67). Knock-out Mäuse von EphB2, EphB3 und EphB4 zeigen Defekte bei der Ausbildung des Blutgefäßsystems. Die embryonale Lethalität der EphB4 -/- Mäuse im Embryonalstadium d14, zeigt die besondere Rolle von EphB4 in diesem Prozeß (Gerety S.S: et al 1999, Mol. Cell 4, 403-414). Eine Modulation dieser Rezeptoren z.B. durch die Inhibition ihrer Kinaseaktivität führt beispielsweise dazu, dass das Tumorwachstum und/oder die Tumormetastasierung entweder durch eine direkte antitumorale oder durch eine indirekte antiangiogene Wirkung unterbunden wird.

Nicht-Rezeptortyrosinkinasen kommen in löslicher Form intrazellulär vor und sind an der Verarbeitung von extrazellulären Signalen (z.B. von Wachstumsfaktoren, Cytokinen, Antikörpern, Adhäsionsmolekülen) innerhalb der Zelle beteiligt. Zu ihnen zählen u.a. die Familien der src(sarcoma)-Kinasen, der Tec(tyrosine kinase expressed in hepatocellular carcinoma)-Kinasen, der Abl(Abelson)-kinasen und der Brk(breast tumor kinase)-Kinasen, sowie die fokale Adhesionskinase (FAK).

Eine veränderte Aktivität dieser Proteintyrosinkinasen kann zu verschiedensten physiologischen Störungen im menschlichen Organismus führen und dadurch z.B. entzündliche, neurologische und onkologische Erkankungen verursachen.

In WO 01/19828 A werden verschiedenste Kinaseinhibitoren offenbart.

In US 2004116388 A werden Triazinverbindungen offenbart, die Rezeptortyrosinkinasen inhibieren.

In WO 03/089434 A werden Imidazo[1,2a]pyrazin-8-yl-amine und in WO 04/00820 A verschiedene aromatische Monozyklen offenbart, die Rezeptortyrosinkinasen inhibieren.

Die DE 24 27 409 A1 beschreibt 9-(substituierte Amino) imidazo[4,5f]-chinoline als wirksame anthelminthische Mittel.

In EP 0 187 705 A2 werden Imidazo[4,5f]-chinoline beschrieben, welche eine immunmodulierende Wirkung bei Infektionskrankheiten aufweisen. In der US 4 716 168 werden auch Imidazo[4,5f]-chinoline mit immunomodulierender Wirkung beschrieben. Ebenso offenbart US 5,506,235 A Imidazo[4,5f]-chinoline mit immunstimulierender Wirkung.

Ferlin M.G., et al 2000, Bioorganic & Med. Chem 8(6), 1415-1422 offenbart Pyrrolo-quinoline mit zellwachstums-inhibierenden Eigenschaften.

In WO 04/006846 A werden verschiedene Chinazolinderivate offenbart, die Rezeptortryrosinkinasen inhibieren.

Unter den Rezeptortyrosinkinaseinhibitoren sind jedoch keine Eph-Rezeptorinhibitoren beschrieben.

Es ist Aufgabe der vorliegenden Erfindung, Verbindungen bereitzustellen, die Rezeptortyrosinkinasen, insbesondere Eph-Rezeptoren, inhibieren.

Die Aufgabe wird gelöst durch Chinolinderivate mit der allgemeinen Formel **A** nach Anspruch 1, die Verwendungen des Chinolinderivats nach den Ansprüchen 11 bis 15, ein Verfahren zur Herstellung des Chinolinderivats nach Anspruch 16 sowie ein das Chinolinderivat enthaltendes Arzneimittel nach Anspruch 17. Vorteilhafte Ausführungsformen sind in den Unteransprüchen angegeben.

Die vorliegende Erfindung betrifft ein Chinolinderivat mit der allgemeinen Formel **A**

Chinolinderivat mit der allgemeinen Formel **A**: 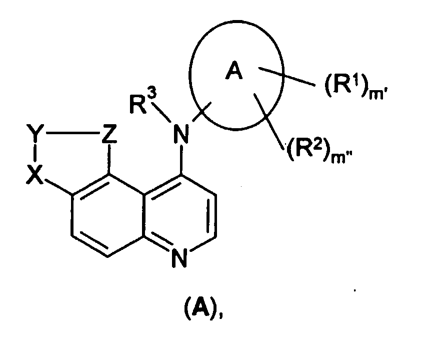 wobei
- A: ausgewählt ist aus der Gruppe, umfassend -C₆-C₁₂-Aryl, -C₅-C₁₈- Heteroaryl, -C₃-C₁₂Cycloalkyl und -C₃-C₁₂-Heterocycloalkyl,
- R¹ und R²: gleich oder verschieden und einfach oder mehrfach unabhängig voneinander ausgewählt sind aus der Gruppe, umfassend Was- serstoff, Hydroxy, Halogen, Nitro, Cyano, -C₁-C₆-Alkyl, -C₁-C₄- Hydroxyalkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, -C₁-C₆- Alkoxy, -C₁-C₆-Alkoxy-C₁-C₆-alkoxy, -C₁-C₆-Alkoxy-C₁-C₆-alkyl, -C₁-C₆-Alkoxy-C₁-C₆-alkoxy-C₁-C₆-alkyl, -(CH₂)ₙ-C₆-C₁₂-Aryl, -(CH₂)ₙ-C₅-C₁₈-Heteroaryl, -(CH₂)ₙ-C₃-C₁₀-Cycloalkyl, -(CH₂)ₙ-C₃- C₁₂-Heterocycloalkyl, -Phenylen-(CH₂)ₚ-R⁶, -(CH₂)ₚPO₃(R⁶)₂, -(CH₂)ₚ-NRR⁶, -(CH₂)ₚ-NR⁴COR⁵, -(CH₂)ₚ-NR⁴CSR⁵, -(CH₂)ₚ- NR⁴S(O)R⁵, -(CH₂)ₚ-NR⁴S(O)₂R⁵, -(CH₂)ₚ-NR⁴CONR⁵R⁶, -(CH₂)ₚ-NR⁴COOR⁵, -(CH₂)ₚ-NR⁴C(NH)NR⁵R⁶, -(CH₂)ₚ-NR⁴CSNR⁵R⁶, -(CH₂)ₚ-NR⁴S(O)NR⁵R⁶, -(CH₂)ₚ- NR⁴S(O)₂NR⁵R⁶, -(CH₂)ₚ-COR⁵, -(CH₂)ₚ-CSR⁵, -(CH₂)ₚ-S(O)R⁵, -(CH₂)ₚ -S(O)(NH)R⁵, -(CH₂)ₚ-S(O)₂R⁵, -(CH₂)ₚ-S(O)₂NR⁵R⁶, - (CH₂)ₚ-SO₂OR⁵, -(CH₂)ₚ-CO₂R⁵, -(CH₂)ₚ-CONR⁵R⁶, -(CH₂)ₚ- CSNR⁵R⁶, -OR⁵, -(CH₂)ₚ-SR⁵ und -CR⁵(OH)-R⁶, wobei -C₁-C₆- Alkyl, -C₂-C₆-Alkenyl, -C₂-C₆-Alkinyl, -C₃-C₁₀-Cycloalkyl, -C₃-C₁₂- Heterocycloalkyl, -C₆-C₁₂-Aryl, -C₅-C₁₈-Heteroaryl und/oder -C₁- C₆-Alkoxy unsubstituiert sind oder einfach oder mehrfach unab- hängig voneinander mit Hydroxy, Halogen, Nitro, Cyano, Phenyl, - NR⁵R⁶, Alkyl und/oder -OR⁵ substituiert sind, wobei das Kohlen- stoffgerüst des -C₃-C₁₀-Cycloalkyls und des -C₁-C₁₀-Alkyls einfach oder mehrfach unabhängig voneinander Stickstoff-, Sauerstoff-, Schwefelatome und/oder C=O-Gruppen und/oder eine oder meh- rere Doppelbindungen enthalten kann, und/oder R¹ und R² optio- nal miteinander eine Brücke aus 3-10 Methyleneinheiten bilden, wobei bis zu zwei Methyleneinheiten mit O, S und/oder -NR⁴ opti- onal ersetzt werden,
- X, Y, Z: gleich oder verschieden und unabhängig voneinander ausgewählt sind aus der Gruppe, umfassend -CR³=, -CR³R⁴-, -C(O)-, -N=, -S-, -O-, -NR³-, -S(O)₂-, -S(O)- und -S(O)NH-, und sich zwischen X, Y und Z Einfach- oder Doppelbindungen befinden,
- R³: Wasserstoff, -C₁-C₁₀-Alkyl oder -C₁-C₁₀-Alkanoyl ist,
- R⁴: Wasserstoff oder -C₁-C₁₀-Alkyl,
- R⁵ und R⁶: gleich oder verschieden und unabhängig voneinander ausgewählt sind aus der Gruppe, umfassend Wasserstoff, -C₁-C₁₀-Alkyl, -C₂- C₁₀-Alkenyl, -C₂-C₁₀-Alkinyl, -C₁-C₆-Alkoxy, -C₃-C₁₀-Cycloalkyl, - C₃-C₁₂-Heterocycloalkyl, -C₆-C₁₂-Aryl und -C₅-C₁₈-Heteroaryl, wo- bei -C₁-C₁₀-Alkyl, -C₂-C₁₀-Alkenyl, -C₂-C₁₀-Alkinyl, -C₁-C₆-Alkoxy, - C₃-C₁₀-Cycloalkyl, -C₃-C₁₂-Heterocycloalkyl, -C₆-C₁₂-Aryl und/oder -C₅-C₁₈-Heteroaryl unsubstituiert sind oder einfach oder mehrfach unabhängig voneinander mit Hydroxy, Halogen, Cyano, Nitro, -OR⁷, -NR⁷R⁸, -C(O)NR⁷R⁸, -C(O)OR⁷ und/oder -C₁-C₆-Alkyl, wo- bei -C₁-C₆-Alkyl unsubstituiert ist oder einfach oder mehrfach un- abhängig voneinander mit Halogen, Hydroxy, Cyano, -NR⁷R⁸, -OR⁷ und/oder Phenyl; und/oder R⁵ und R⁶ optional miteinander eine Brücke aus 3-10 Methyleneinheiten bilden, wobei bis zu zwei Methyleneinheiten mit O, S und/oder NR⁴ optional ersetzt werden;
- R⁷, R⁸: gleich oder verschieden und unabhängig voneinander ausgewählt sind aus der Gruppe, umfassend Wasserstoff, -C₁-C₄-Alkyl, -C₆- C₁₂-Aryl and -C₅-C₁₈-Heteroaryl, wobei Alkyl, Aryl, Heteroaryl un- substituiert ist oder einfach oder mehrfach unabhängig voneinan- der mit Halogen und/oder Alkoxy, oder R⁷ und R⁸ optional mitein- ander eine Brücke aus 3-10 Methyleneinheiten bilden, wobei bis zu zwei Methyleneinheiten mit O, S und/oder -NR⁴ optional ersetzt werden;
- m', m": = unabhängig voneinander 0 - 4,
- n: =1-6,
- p: = 0 - 6, sowie
deren N-Oxide, Solvate, Hydrate, Stereoisomere, Diastereomere, Enantiomere und Salze.

Sofern X,Y,Z unabhängig voneinander ein, zwei oder drei N bedeuten, gilt die Maßgabe, dass
1. das Skelett in der Teilgruppierung X-Y-Z nicht N-CH-N, CH-N-N oder N-N-N ist und
2. X nicht NH ist, wenn Y und Z gleichzeitig jeweils CH sind.

Es wurde gefunden, dass die erfindungsgemäßen Verbindungen Rezeptortyrosinkinasen, insbesondere Eph-Rezeptoren, inhibieren können.

Die im letztgenannten Abschnitt unter 1. genannte Teilgruppierung CH-N-N und N-N-N wurde in der oben genannten US 5,506,235 A beschrieben. Substanzen mit dieser Teilgruppierung haben gemäß der US 5,506,235 A immunstimulierender Wirkung. Die im letztgenannten Abschnitt unter 1. genannte Teilgruppierung N-CH-N werden in der DE 24 27 409 A1, der EP 0 187 705 A2 oder der US 4 716 168 beschrieben. Substanzen mit dieser Teilgruppierung haben gemäß der DE 24 27 409 A1 eine anthelminthische Wirkung und gemäß der EP 0 187 705 A2 und der US 4 716 168 eine immunmodulierende Wirkung. Verbindungen mit zellwachstums-inhibierenden Eigenschaften die unter die im letztgenannten Abschnitt unter 2. aufgeführten Kriterien fallen, werden in Ferlin M.G., et al 2000, Bioorganic & Med. Chem 8(6), 1415-1422 beschrieben. In allen in diesem Absatz beschriebenen Dokumenten werden jedoch keine Eph-Rezeptorinhibitoren offenbart.

Unter Alkyl ist jeweils ein geradkettiger oder verzweigter Alkylrest, wie beispielsweise Methyl, Ethyl, Propyl, Isopropyl, Butyl, Isobutyl, sek. Butyl, *tert-*Butyl, Pentyl, Isopentyl, Hexyl, Heptyl, Octyl, Nonyl und Decyl, zu verstehen.

Unter Alkoxy ist jeweils ein geradkettiger oder verzweigter Alkoxyrest, wie beispielsweise Methyloxy, Ethyloxy, Propyloxy, Isopropyloxy, Butyloxy, Isobutyloxy, sek. Butyloxy, Pentyloxy, Isopentyloxy, Hexyloxy, Heptyloxy, Octyloxy, Nonyloxy oder Decyloxy zu verstehen.

Die Alkenyl-Substituenten sind jeweils geradkettig oder verzweigt, wobei beispielsweise folgenden Reste gemeint sind: Vinyl, Propen-1-yl, Propen-2-yl, But-1-en-1-yl, But-1-en-2-yl, But-2-en-1-yl, But-2-en-2-yl, 2-Methyl-prop-2-en-1-yl, 2-Methyl-prop-1-en-1-yl, But-1-en-3-yl, But-3-en-1-yl, Allyl.

Unter Alkinyl ist jeweils ein geradkettiger oder verzweigter Alkinyl-Rest zu verstehen, der zwei bis sechs, bevorzugt zwei bis vier C-Atome enthält. Beispielsweise sind die folgenden Reste geeignet: Ethinyl, Propin-1-yl, Propin-3-yl, But-1-in-1-yl, But-1-in-4-yl, But-2-in-1-yl, But-1-in-3-yl.

Als Cycloalkyl, welches ein oder mehrere Heteroatome wie Schwefel, Stickstoff oder Sauerstoff enthalten kann, seien z.B. genannt: Oxiranyl, Oxethanyl, Aziridinyl, Azetidinyl, Tetrahydrofuranyl, Pyrrolidinyl, Dioxolanyl, Imidazolidinyl, Pyrazolidinyl, Dioxanyl, Piperidinyl, Morpholinyl, Dithianyl, Thiomorpholinyl, Piperazinyl, Trithianyl, Chinuclidinyl.

Unter Cycloalkyl sind monocyclische Alkylringe wie Cyclopropyl, Cyclobutyl, Cyclopentyl, Cyclohexyl oder Cycloheptyl, aber auch bicyclische Ringe oder tricyclische Ringe wie zum Beispiel Adamantanyl zu verstehen. Die Cycloalkylringe können unsubstituiert oder ein- oder mehrfach substituiert sein. Cycloalkyle gemäß dieser Erfindung enthalten C₃-C₁₂ Kohlenwasserstoffatomen, bevorzugt sind Cycloalkyle mit C₃-C₁₀-Kohlenwasserstoffatomen und besonders bevorzugt sind Cycloalkyle mit C₃-C₆-Kohlenwasserstoffatomen .

Ein Arylrest hat jeweils 6 - 12 Kohlenstoffatome. Der Rest kann mono- oder bicyclisch, beispielsweise Naphthyl, Biphenyl und insbesondere Phenyl, sein.

Der Heteroarylrest umfaßt ein aromatisches Ringsystem, welches jeweils 5 -18 Ringatome, bevorzugt 5 bis 10 Ringatome und besonders bevorzugt 5 bis 7 Ringatome enthält und anstelle des Kohlenstoffs ein oder mehrere gleiche oder verschiedene Heteroatome aus der Gruppe Sauerstoff, Stickstoff oder Schwefel. Der Rest kann mono-, bi- oder tricyclisch und zusätzlich jeweils benzokondensiert sein. Es sind allerdings nur diejenigen Kombinationen gemeint, die aus Sicht eines Fachmanns sinnvoll sind, insbesondere in Bezug auf die Ringspannung.

Die Heteroarylringe können unsubstituiert oder ein- oder mehrfach substituiert sein. Beispielhaft seien genannt: Thienyl, Furanyl, Pyrrolyl, Oxazolyl, Thiazolyl, Imidazolyl, Pyrazolyl, Isoxazolyl, Isothiazolyl, Oxadiazolyl, Triazolyl, Thiadiazolyl, Pyridyl, Pyridazinyl, Pyrimidinyl, Pyrazinyl, Triazinyl sowie Benzoderivate dieser Reste, wie z. B. 1,3-Benzodioxolyl, Benzofuranyl, Benzothienyl, Benzoxazolyl, Benzimidazolyl, Indazolyl, Indolyl, Isoindolyl, Oxepinyl, Azocinyl, Indolizinyl, Indolyl, Isoindolyl, Indazolyl, Benzimidazolyl, Purinyl, Chinolinyl, Isochinolinyl, Cinnolinyl, Phthalazinyl, Chinazolinyl, Chinoxalinyl, Naphthyridinyl, Pteridinyl, Carbazolyl, Acridinyl, Phenazinyl, Phenothiazinyl, Phenoxazinyl, Xanthenyl etc.

Unter Halogen ist jeweils Fluor, Chlor, Brom oder Jod zu verstehen.

C₃-C₁₂-Heterocycloalkyl steht für einen 3 -12 Kohlenstoffatome, bevorzugt für einen 3 bis 10 Kohlenstoffatome und besonders bevorzugt für einen 3 bis 6 Kohlenstoffatome umfassenden Alkylring, der durch mindestens eins der folgenden Atome Stickstoff, Sauerstoff und/ oder Schwefel im Ring unterbrochen ist und der gegebenenfalls durch ein oder mehrere, gleich oder verschiedene - (CO)-, -SO- oder -SO₂- Gruppen im Ring unterbrochen sein kann und gegebenenfalls ein oder mehrere Doppelbindungen im Ring enthält. Es sind allerdings nur diejenigen Kombinationen gemeint, die aus Sicht eines Fachmanns sinnvoll sind, insbesondere in Bezug auf die Ringspannung. C₃-C₁₂-Herterocycloalkyle gemäß dieser Erfindung sind monocyclisch, aber auch bicyclisch oder tricyclisch. Als monocyclische Heterocyclyle seien z. B. genannt: Oxiranyl, Oxethanyl, Aziridinyl, Azetidinyl, Tetrahydrofuranyl, Pyrrolidinyl, Dioxolanyl, Imidazolidinyl, Pyrazolidinyl, Dioxanyl, Piperidinyl, Morpholinyl, Dithianyl, Thiomorpholinyl, Piperazinyl, Trithianyl, Chinuclidinyl etc.

So wie in dieser Anmeldung verwendet, bezeichnet "C₁-C₁₀", zum Beispiel im Zusammenhang mit der Definition von "C₁-C₁₀-Alkyl" eine Alkylgruppe mit einer endlichen Anzahl von 1 bis 10 Kohlenstoffatomen, d.h. 1,2,3,4,5,6,7,8,9 oder 10 Kohlenstoffatome. Weiter wird die Definition "C₁-C₁₀" so interpretiert, dass jeder mögliche Teilbereich, wie beispielsweise, C₁-C_{10 ,} C₂-C_{9 ,} C₃-C_{8 ,} C₄-C_{7 ,} C₅-C_{6,} C₁-C_{2 ,} C₁-C_{3 ,} C₁-C_{4 ,} C₁-C_{5 ,} C₁-C_{6 ,} C₁-C_{7 ,} C₁-C_{8 ,} C₁-C_{9 ,} C₁-C_{10 ,} bevorzugt C₁-C_{2 ,} C₁-C_{3 ,} C₁-C_{4 ,} C₁-C_{5 ,} C₁-C_{6 ;} bevorzugt C₁-C₄ in der Definition mitbeinhaltet ist.

Analog dazu, bezeichnet "C₂-C₁₀", zum Beispiel in Zusammenhang mit der Definition von "C₂-C₁₀-Alkenyl" und "C₂-C₁₀-Alkinyl" eine Alkenylgruppe oder Alkinylgruppe mit einer endlichen Anzahl von 2 bis 10 Kohlenstoffatomen, d.h. 2,3,4,5,6,7,8,9 oder 10 Kohlenstoffatome. Die Definition "C₂-C₁₀" wird so interpretiert, dass jeder mögliche Teilbereich, wie beispielsweise C₂-C_{10 ,} C₃-C_{9 ,} C₄-C_{8 ,} C₅-C_{7 ,} C₂-C_{3 ,} C₂-C_{4 ,} C₂-C_{5 ,} C₂-C_{6,} C₂-C_{7 ,} C₂-C_{8 ,} C₂-C_{9 ,} bevorzugt C₂-C₄ in der Definition mitbeinhaltet ist.

Weiter bezeichnet C₁-C₆", zum Beispiel in Zusammenhang mit der Definition von "C₁-C₆-Alkoxy" eine Alkoxygruppe mit einer endlichen Anzahl von 1 bis 6 Kohlenstoffatomen, d.h. 1,2,3,4,5 oder 6 Kohlenstoffatome. Die Definition "C₁-C₆" wird so interpretiert, dass jeder mögliche Teilbereich, wie beispielsweise C₁-C_{6,} C₂-C_{5 ,} C₃-C_{4 ,} C₁-C_{2 ,} C₁-C_{3 ,} C₁-C_{4 ,} C₁-C_{5 ,} C₁-C_{6 ;} bevorzugt C₁-C₄, in der Definition mitbeinhaltet ist.

Alle hier nicht explizit aufgeführten Bereichsangaben der Anmeldung sind analog wie die oben exemplarisch genannten Bereiche "C₁-C₁₀", "C₂-C₁₀" und "C₁-C₆" definiert.

Unter Isomeren sind chemische Verbindungen der gleichen Summenformel, aber unterschiedlicher chemischer Struktur, zu verstehen. Es werden im Allgemeinen Konstitutionsisomere und Stereoisomere unterschieden. Konstitutionsisomere besitzen die gleiche Summenformel, unterscheiden sich jedoch durch die Verknüpfungsweise ihrer Atome oder Atomgruppen. Hierzu zählen funktionelle Isomere, Stellungsisomere, Tautomere oder Valenzisomere. Stereoisomere haben grundsätzlich die gleiche Struktur (Konstitution) und damit auch die gleiche Summenformel, unterscheiden sich aber durch die räumliche Anordnung der Atome. Im Allgemeinen werden Konfigurationsisomere und Konformationsisomere unterschieden. Konfigurationsisomere sind Stereoisomere, die sich nur durch Bindungsbruch ineinander überführen lassen. Hierzu zählen Enantiomere, Diastereomere und E / Z (cis / trans) Isomere. Enantiomere sind Stereoisomere, die sich wie Bild und Spiegelbild zueinander verhalten und keine Symmetrieebene aufweisen. Alle Stereoisomere, die keine Enantiomere sind, bezeichnet man als Diastereomere. Ein Spezialfall sind E / Z (cis / trans) Isomere an Doppelbindungen. Konformationsisomere sind Stereoisomere, die sich durch die Drehung von Einfachbindungen ineinander überführen lassen. Zur Abgrenzung der Isomerie-Arten voneinander siehe auch die IUPAC Regeln Sektion E (Pure Appl. Chem. 45, 11-30, 1976).

Die erfindungsgemäßen Chinolinderivate mit der allgemeinen Formel **A** beinhalten auch die möglichen tautomeren Formen und umfassen die E- oder Z-Isomeren oder, falls ein chirales Zentrum vorhanden ist, auch die Racemate und Enantiomeren. Hierunter sind auch Doppelbindungsisomeren zu verstehen.

Die erfindungsgemäßen Chinolinderivate können auch in Form von Solvaten, insbesondere von Hydraten vorliegen, wobei die erfindungsgemäßen Verbindungen demgemäß polare Lösungsmittel, insbesondere von Wasser, als Strukturelement des Kristallgitters der erfindungsgemäßen Verbindungen enthalten. Der Anteil an polarem Lösungsmittel, insbesondere Wasser kann in einem stöchiometrischen oder auch unstöchiometrischen Verhältnis vorliegen. Bei stöchiometrischen Solvaten, Hydraten spricht man auch von Hemi-, (Semi-), Mono-, Sesqui-, Di-, Tri-, Tetra-, Penta-, usw. Solvaten oder Hydraten.

N-Oxide bedeutet, dass mindestens ein Stickstoff der erfindungsgemäßen Verbindungen der allgemeinen Formel **A** oxidiert sein kann.

Ist eine saure Funktion enthalten, sind als Salze die physiologisch verträglichen Salze organischer und anorganischer Basen geeignet, wie beispielsweise die gut löslichen Alkali- und Erdalkalisalze sowie Salze von N-Methyl-glukamin, Dimethyl-glukamin, Ethyl-glukamin, Lysin, 1,6-Hexadiamin, Ethanolamin, Glukosamin, Sarkosin, Serinol, Tris-hydroxy-methyl-amino-methan, Aminopropandiol, Sovak-Base, 1-Amino-2,3,4-butantriol.

Ist eine basische Funktion enthalten, sind die physiologisch verträglichen Salze organischer und anorganischer Säuren geeignet wie Salzsäure, Schwefelsäure, Phosphorsäure, Zitronensäure, Weinsäure u.a.

Funktionelle Gruppen können gegebenenfalls durch Schutzgruppen während der Reaktionsequenz geschützt werden. Solche Schutzgruppen können unter anderem Ester, Amide, Ketale/Acetale, Nitrogruppen, Carbamate, Alkylether, Allylether, Benzylether oder Silylether sein. Als Bestandteil von Silylethern können unter anderem Verbindungen, wie z.B. Trimethylsilyl (TMS), *tert*-Butyldimethylsilyl (TBDMS), *tert*-Butyl-diphenylsilyl (TBDPS), Triethylsilyl (TES), etc., vorkommen. Deren Herstellung wird im experimentellen Teil beschrieben.

Bevorzugt sind Chinolinderivate mit der vorgenannten Allgemeinen Formel **A**, mit der Maßgabe, dass wenn X,Y,Z unabhängig voneinander ein, zwei oder drei N bedeuten,
1. das Skelett in der Teilgruppierung X-Y-Z nicht N-N-CH, N-CH-N, CH-N-N oder N-N-N ist und
2. X nicht NH ist, wenn Y und Z gleichzeitig jeweils CH sind.

Bevorzugt sind Chinolinderivate mit der vorgenannten allgemeinen Formel **A**, in denen:
- R¹ und R²: gleich oder verschieden und einfach oder mehrfach unabhängig voneinander ausgewählt sind aus der Gruppe, umfassend Was- serstoff, Hydroxy, Halogen, Nitro, Cyano, -C₁-C₆-Alkyl, -C₁-C₄- Hydroxyalkyl, -C₆-C₁₂-Aryl, -C₁-C₆-Alkoxy, -NR⁵R⁶, -NR⁴COR⁵, -NR⁴S(O)R⁵, -NR⁴S(O)₂R⁵, -NR⁴CONR⁵R⁶, -NR⁴S(O)NR⁵R⁶, -NR⁴S(O)₂NR₅R⁶, -COR⁵, COOR⁵, -S(O)R⁵, -S(O)(NH)R⁵, - S(O)₂R⁵, -S(O)₂NR⁵R⁶, -CO₂R⁵, -CONR⁵R⁶, -OR⁵ und -CR⁵(OH)- R⁶, und
- m', m": = unabhängig voneinander 0 - 3.

Vorzugsweise ist R³ in der allgemeinen Formel **A** Wasserstoff.

Vorzugsweise ist A in der allgemeinen Formel **A** Phenyl.

Besonders bevorzugt sind Verbindungen der allgemeinen Formel **A** in den der Ring A Phenyl und R¹ und R² gleich oder verschieden und einfach oder mehrfach unabhängig voneinander ausgewählt sind aus der Gruppe, umfassend Wasserstoff, Hydroxy, Halogen, Nitro, Amino, Cyano, -C₁-C₆-Alkyl, -C₁-C₄-Hydroxyalkyl, -C₁-C₆-Alkoxy, -NH-C(O)-NH-Aryl, -C₁-C₄-Alkyl-CO-NH-, -COOR⁵ und vorzugsweise -COOR^{N}, wobei R^{N} für H, Alkyl, Alkenyl, Cycloalkyl oder Aryl steht, -CR⁵(OH)-R⁶ und -CONH₂, und
m', m" = unabhängig voneinander 0 - 3.

Ganz besonders bevorzugt sind dabei Verbindungen in denen R¹ und R² gleich oder verschieden und einfach oder mehrfach unabhängig voneinander ausgewählt sind aus der Gruppe, umfassend Wasserstoff, Hydroxy, Halogen, Nitro, Amino, Cyano, -CH₃, -C₂H₅, CH₃O-, C₂H₅O-, HOCH₂-, CH₃CONH-, -NH-C(O)-NH-Phenyl, -COOH und -CONH₂.

Weiterhin sind Chinolinderivate mit der allgemeinen Formel **A** bevorzugt, bei denen X,Y und Z unabhängig voneinander ausgewählt sind aus der Gruppe, umfassend -CR⁴=, -CR⁴R⁵-, -C(O)-, -N=, -S-, -O-, -NR⁴-, -S(O)₂-, -S(O)- und -S(O)NH-, wobei N, S oder O im Ring nicht mehrfach vorkommen. In diesem Falle sind vorzugsweise der Ring A Phenyl und m' und m" = 0 - 2.

Bevorzugt sind weiterhin Verbindungen der allgemeinen Formel A, bei denen X, Y und Z für -S(O)₂-, -S-, -NH-, -CH=, -C(CH₃)= und/oder -CH₂-stehen.

Das Skelett in der Teilgruppierung X-Y-Z in dem Chinolinderivat mit der allgemeinen Formel **A** ist ganz besonders bevorzugt ausgewählt aus der Gruppe, umfassend -S-CH=CH-, -S-C(C₁-C₆-Alkyl)=N- und bevorzugt -S-C(C₁-C₃-Alkyl)=N- und noch bevorzugter -S-C(CH₃)=N-, -S(O)₂-CH₂-CH₂- und -CH=CH-S-.

Am meisten bevorzugt sind folgende Verbindungen:
1) 4-Methyl-3-(thieno[3,2-f]chinolin-9-ylamino)-phenol
2) 4-Methyl-3-(2-methyl-thiazolo[4,5-f]chinolin-9-ylamino)-phenol
3) 4-Methyl-3-(thieno[2,3-f]chinolin-9-yl-amino)phenol
4) 3-(3,3-Dioxo-2,3-dihydro-1H-3λ⁶-thieno[3,2-f]chinolin-9-ylamino)-4-methylphenol
5) 3-(3,3-Dioxo-2,3-dihydro-1H-3λ⁶-thieno[3,2-f]quinolin-9-ylamino)-phenol
6) 4-(3,3-Dioxo-2,3-dihydro-1H-3λ⁶-thieno[3,2-f]chinolin-9-ylamino)-3-methylphenol
7) 2-(3,3-Dioxo-2,3-dihydro-1H-3λ⁶-thieno[3,2-f]chinolin-9-ylamino)-phenol
8) 4-(3,3-Dioxo-2,3-dihydro-1H-3λ⁶-thieno[3,2-f]quinolin-9-ylamino)-phenol
9) [3-(3,3-Dioxo-2,3-dihydro-1H-3λ⁶-thieno[3,2-f]quinolin-9-ylamino)phenyl]-methanol
10) 3-(3,3-Dioxo-2,3-dihydro-1H-3λ⁶-thieno[3,2-f]chinolin-9-ylamino)-benzoesäure
11) 3-(3,3-Dioxo-2,3-dihydro-1H-3λ⁶-thieno[3,2-f]chinolin-9-ylamino)-benzamid
12) (3,3-Dioxo-2,3-dihydro-1H-3λ⁶-thieno[3,2-f]quinolin-9-yl)-(3-methoxyphenyl)amin
13) N-[3-(3,3-Dioxo-2,3-dihydro-1H-3λ⁶-thieno[3,2-f]chinolin-9-ylamino)-phenyl]-acetamid
14) 3-(3,3-Dioxo-2,3-dihydro-1H-3λ⁶-thieno[3,2-f]chinolin-9-ylamino)-5-methoxyphenol
15) 5-(3,3-Dioxo-2,3-dihydro-1H-3λ⁶-thieno[3,2-f]chinolin-9-ylamino)-2-methylphenol
16) 3-(3,3-Dioxo-2,3-dihydro-1H-3λ⁶-thieno[3,2-f]chinolin-9-ylamino)-2-methylphenol
17) 3-(3,3-Dioxo-2,3-dihydro-1H-3λ⁶-thieno[3,2-f]chinolin-9-ylamino)-5-methylPhenol
18) 4-Chloro 3-(3,3-dioxo-2,3-dihydro-1H-3λ⁶-thieno[3,2-f]chinolin-9-ylamino)-5-methyl-phenol
19) 2-(3,3-Dioxo-2,3-dihydro-1H-3λ⁶-thieno[3,2-f]chinolin-9-ylamino)-4-methoxyPhenol
20) (3,3-Dioxo-2,3-dihydro-1H-3λ⁶-thieno[3,2-f]-chinolin-9-yl)-(2-methyl-5-nitrophenyl)-amin
21) [3-(3,3-Dioxo-2,3-dihydro-1H-3λ⁶-thieno[3,2-f]quinolin-9-ylamino)-4-methoxy-phenyl]-methanol
22) 1-[3-(3,3-Dioxo-2,3-dihydro-1H-3λ⁶-thieno[3,2-f]chinolin-9-ylamino)-phenyl]-3-phenyl-harnstoff
23) 1-[4-(3,3-Dioxo-2,3-dihydro-1H-3λ⁶-thieno[3,2-f]quinolin-9-ylamino)-phenyl]-3-phenyl-harnstoff
24) (3,5-Dimethoxy-phenyl)-(3,3-dioxo-2,3-dihydro-1H-3λ⁶-thieno[3,2-f]chinolin-9-yl)-amin
25) (3,3-Dioxo-2,3-dihydro-1H-3λ⁶-thieno[3,2-f]-chinolin-9-yl)-(3,4,5-trimethoxyphenyl)-amin
26) N³-(3,3-Dioxo-2,3-dihydro-1H-3λ⁶-thieno[3,2-f]quinolin-9-yl)-4-methylphenyl-1,3-diamin
27) 1-[3-(3,3-Dioxo-2,3-dihydro-1H-3λ⁶-thieno[3,2-f]chinolin-9-ylamino)-4-methyl-phenyl]-3-phenyl-harnstoff

Die erfindungsgemäßen Chinolinderivate mit der allgemeinen Formel **A** inhibieren Rezeptortyrosinkinasen, insbesondere Eph Kinasen, worauf auch deren Wirkung zum Beispiel bei der Behandlung von Erkrankungen, in denen die Angiogenese, Lymphangiogenese oder Vaskulogenese eine Rolle spielen, bei Erkrankungen der Blutgefäße, Erkrankungen, die durch eine Hyperproliferation von Körperzellen hervorgerufen werden oder chronischen oder akuten neurodegenerativen Erkrankungen. Die vorliegenden Chinolinderivate mit der allgemeinen Formel A können demnach als Arzneimittel verwendet werden.

Behandlungen werden bevorzugt am Menschen, aber auch an verwandten Säugetierspezies wie z.B. Hund und Katze durchgeführt.

Angiogene und/oder vaskulogene Erkrankungen können behandelt werden, indem das Wachstum der Blutgefäße inhibiert (antiangiogen) oder gefördert (proangiogen) wird. Antiangiogene Verwendungen erfolgen z.B. bei der Tumorangiogenese, der Endometriose, bei diabetisch-bedingten oder anderen Retinopathien oder bei altersbedingter Degeneration der Makula. Proangiogene Verwendungen erfolgen z.B. bei Herzinfarkt oder akut neurodegenerativen Erkrankungen durch Ischämien des Gehirns oder Neurotraumata.

Unter Blutgefäßerkrankungen sind Stenosen, Arteriosklerosen, Restenosen oder Entzündungskrankheiten, wie rheumatische Arthritis, zu verstehen.

Unter hyperproliferativen Erkrankungen, sind solide Tumore, nicht-solide Tumore oder nicht-cancerogene Zellhyperproliferation in der Haut zu verstehen, wobei unter soliden Tumoren unter anderen Mamma-, Colon-, Nieren-, Lungen- und/oder Gehirntumore zu verstehen sind. Unter nicht-soliden Tumoren sind unter anderem Leukämien zu verstehen und unter nicht-cancerogener Zellhyperproliferation in der Haut unter anderem Psoriasis, Ekzeme, Skleroderma oder benigne Hypertrophie der Prostata.

Unter chronisch neurodegenerativen Erkrankungen sind u.a. Huntington's Erkrankung, amyotrophe Lateralsklerose, Parkinsonsche Erkrankung, AIDS induzierte Dementia oder Alzheimer'sche Erkrankung zu verstehen.

Verwendung der Chinolinderivate mit der allgemeinen Formel **A** können ebenfalls für diagnostische Zwecke *in vitro* oder *in vivo* zur Identifizierung von Rezeptoren in Geweben mittels Autoradioagraphie und/oder PET verwendet werden.

Insbesondere können die Substanzen für diagnostische Zwecke auch radiomarkiert sein.

Zur Verwendung der erfindungsgemäßen Chinolinderivate als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe, wie Konservierungs-, Stabilisierungs-, Netzmittel oder Emulgatoren; Salze zur Veränderung des osmotischen Drucks oder Puffer.

Diese pharmazeutischen Präparate sind ebenfalls Gegenstand der vorliegenden Erfindung.

Für die parenterale Anwendung sind insbesondere Injektionslösungen oder Suspensionen, insbesondere wässrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposomen oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die enteralen, parenteralen und oralen Applikationen sind ebenfalls Gegenstand der vorliegenden Erfindung.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5-1.000 mg, wobei die Dosis als einmal zu verabreichende Einzeldosis oder unterteilt in zwei oder mehreren Tagesdosen gegeben werden kann.

Ebenfalls Gegenstand der vorliegenden Erfindung sind Arzneimittel zur Behandlung der oben aufgeführten Erkrankungen, die mindestens ein Chinolinderivat mit der allgemeinen Formel **A** enthalten, wobei die Arzneimittel gegebenenfalls geeignete Formulierungs- und Trägerstoffen enthalten können.

Soweit die Herstellung der Ausgangsverbindungen nicht beschrieben wird, sind diese dem Fachmann bekannt oder analog zu bekannten Verbindungen oder hier beschriebenen Verfahren herstellbar. Es ist ebenfalls möglich, alle hier beschriebenen Umsetzungen in Parallel-Reaktoren oder mittels kombinatorischer Arbeitstechniken durchzuführen.

Die Isomerengemische können nach üblichen Methoden wie beispielsweise Kristallisation, Chromatographie oder Salzbildung in die Enantiomeren bzw. E/Z-Isomeren aufgetrennt werden.

Die Herstellung der Salze erfolgt in üblicher Weise, indem man eine Lösung der Verbindung mit der allgemeinen Formel A mit der äquivalenten Menge oder einem Überschuss einer Base oder Säure, die gegebenenfalls in Lösung ist, versetzt und den Niederschlag abtrennt oder in üblicher Weise die Lösung aufarbeitet.

Ebenfalls Gegenstand der vorliegenden Erfindung ist das Verfahren zur Herstellung der erfindungsgemäßen Chinolinderivate.

Die für die Herstellung der erfindungsgemäßen Chinolinderivate mit der allgemeinen Formel **A** vorzugsweise verwendeten Zwischenprodukte sind folgende Verbindungen mit den allgemeinen Formeln **I** bis **VI.**

### Herstellung der erfindungsgemäßen Verbindungen

### Verfahrensvariante 1

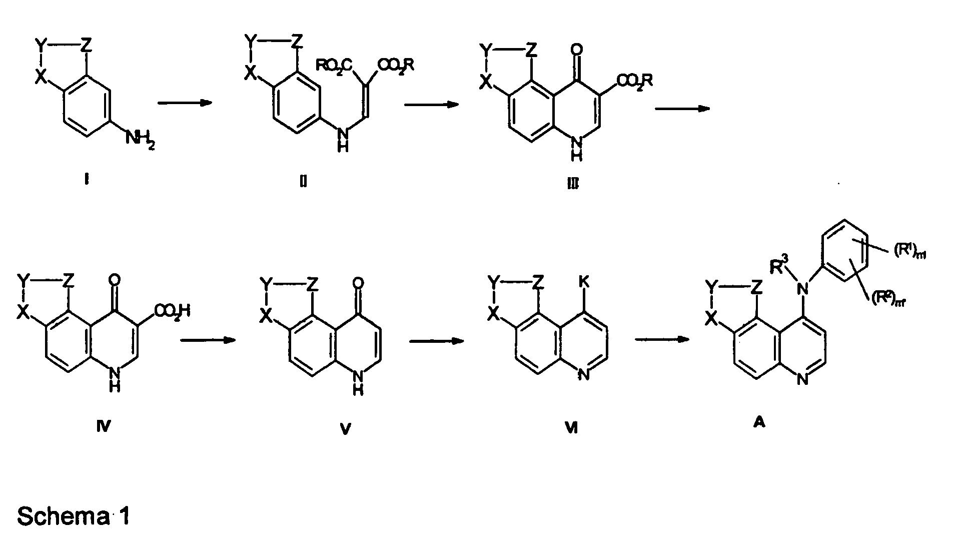

Erfindungsgemäße Chinolinderivate mit der allgemeinen Formel **A** können zum Beispiel auf dem im Schema 1 gezeigten Weg hergestellt werden, worin der Rest K beispielsweise Halogen oder -OS(O)₂CₙF₂ₙ₊₁ mit n= 1-3 und der Rest R Methyl oder Ethyl sein kann und die Reste X, Y und Z dieselbe Bedeutung haben wie in der allgemeinen Formel **A**. Die benötigten Ausgangsmaterialien sind entweder kommerziell erhältlich oder werden nach in der Literatur bekannten Verfahren bzw. in Analogie zu in der Literatur bekannten Verfahren hergestellt.

Durch Addition einer Verbindung mit der allgemeinen Formel I an ein Dialkylalkoxymethylenmalonat, z.B. Diethylethoxymethylenmalonat, werden Verbindungen mit der allgemeinen Formel **II** gebildet. Diese Verbindungen werden dann vorzugsweise unter thermischen Bedingungen zu Verbindungen mit der allgemeinen Formel **III** zyklisiert. Bei diesen Zyklisierungen können auch Säuren oder Lewis-Säuren zum Einsatz kommen. Anschließend wird der Ester verseift, wobei Verbindungen mit der allgemeinen Formel **IV** erhalten werden, die dann vorzugsweise unter thermischen Bedingungen decarboxyliert werden, wobei Verbindungen mit der allgemeinen Formel **V** entstehen. Alternativ kann auch eine direkte Decarboxylierung der Alkylester mit der allgemeinen Formel **III** durchgeführt werden. Neben den erwähnen thermischen Bedingungen können auch andere in der Literatur bekannte Verfahren zur Decarboxylierung sowohl ausgehend von Verbindungen mit der allgemeinen Formel **III** sowie ausgehend von Verbindungen mit der allgemeinen Formel **IV** zum Einsatz kommen. Verbindungen mit der allgemeinen Formel **VI** werden dann z.B durch Umsetzung mit Thionylchlorid (für K=Cl) oder Perfluoralkylsulfonsäureanhydriden (für K= Perfluoralkylsulfonyl) hergestellt. Verbindungen mit der allgemeinen Formel **A** können dann durch Addition von Aminen ((R¹)_{m'},(R²)ₘ-ArNR³H) aus Verbindungen der allgemeinen Formel **VI** hergestellt werden, wobei die Reste X, Y und Z gegebenenfalls weiter modifiziert werden können. In den Zwischenstufen evtl. enthaltene funktionelle Gruppen, wie Carbonylgruppen, Hydroxygruppen oder Aminogruppen können zwischenzeitlich nach bekannten Verfahren mit Schutzgruppen geschützt werden.

Im Folgenden werden Beispiele für erfindungsgemäße Ringsysteme entsprechend der allgemeinen Formel **A** angegeben:

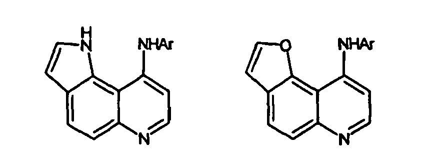

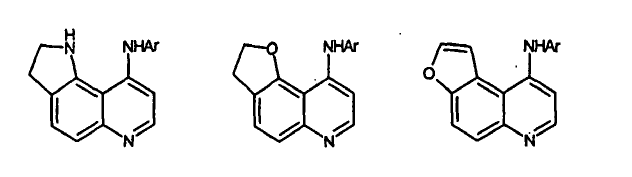

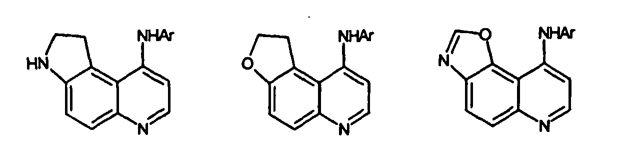

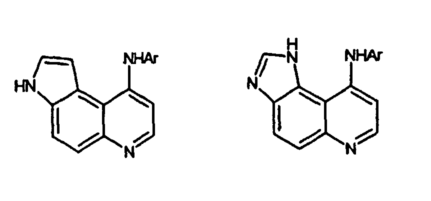

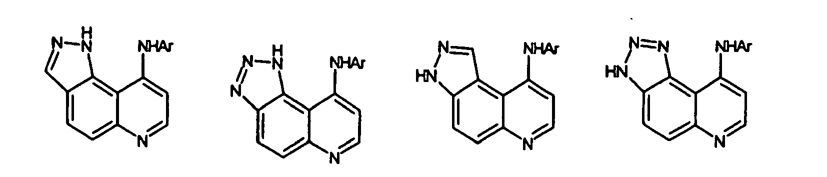

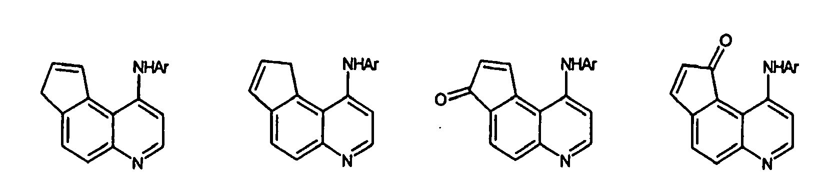

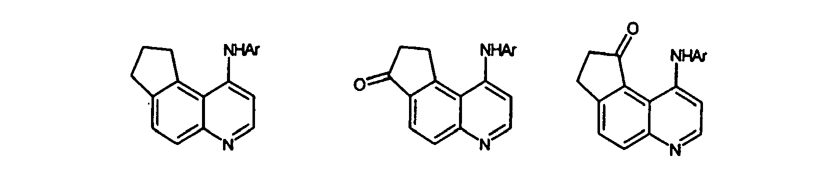

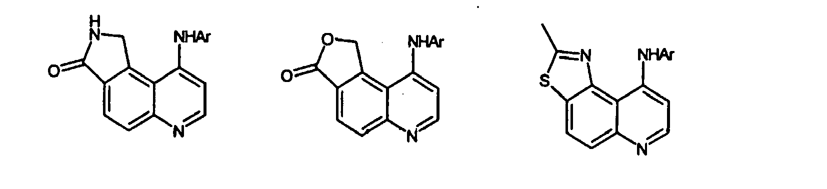

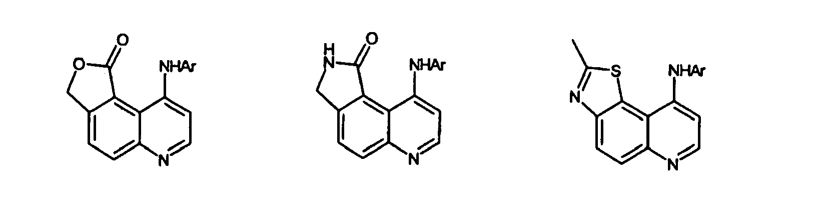

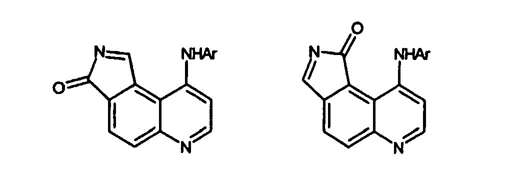

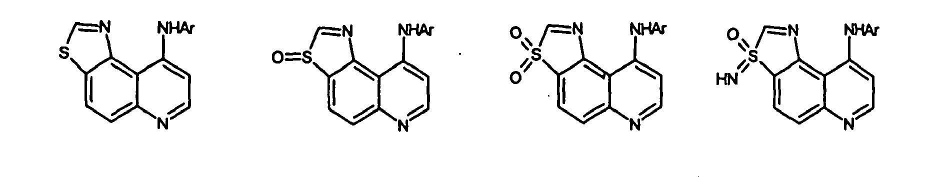

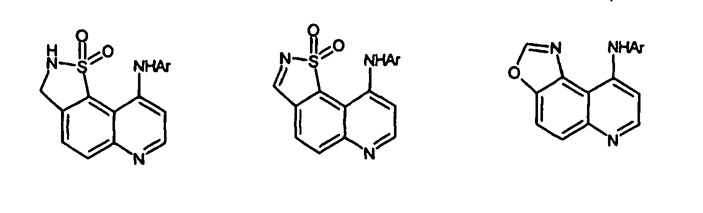

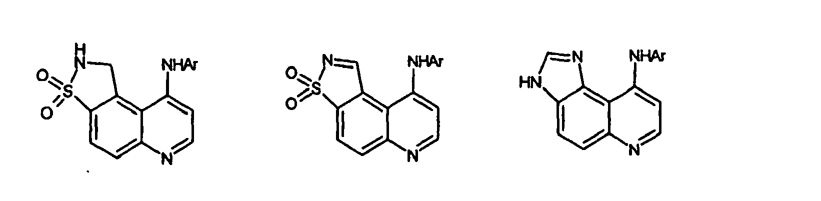

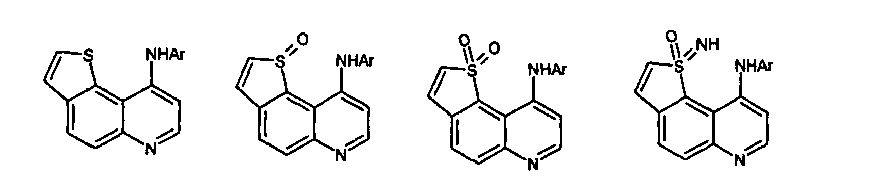

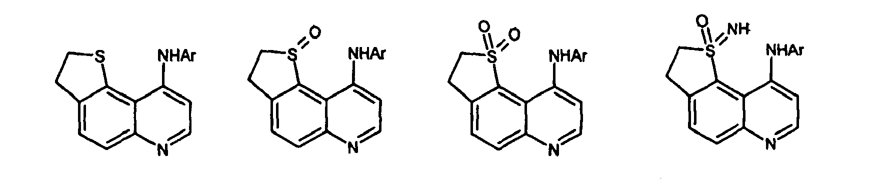

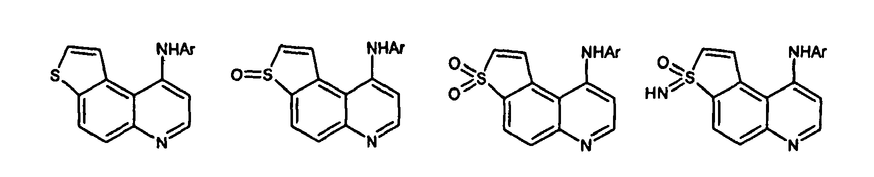

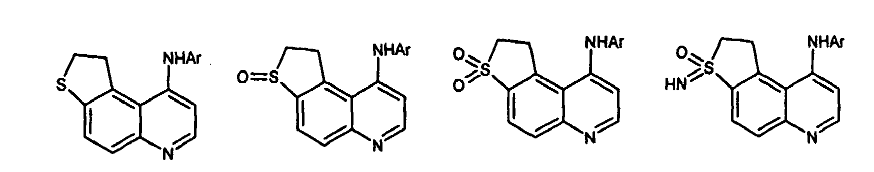

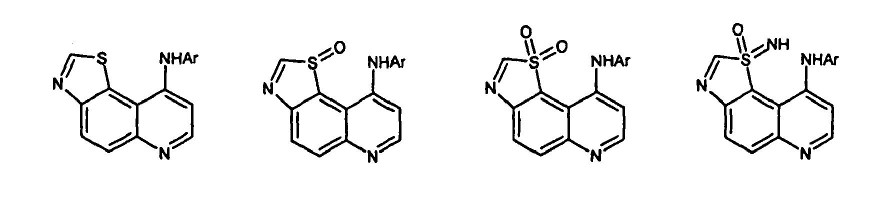

Entsprechend der allgemeinen Formel **A** kann an Stelle von -N= und -NH- in den vorgenannten Beispielen auch -NR⁴- im füngliedrigen Heterozyklus stehen, wobei R⁴ beispielsweise C₁-C₁₀-Alkyl oder C₁-C₁₀-Alkanoyl ist. Im Falle von -N= würde die von N ausgehende Doppelbindung dann entfallen.

Sofern X,Y und/oder Z in dem füngliedrigen Ring Kohlenstoff ist, können diese ebenfalls einfach oder mehrfach substituiert sein, beispielsweise Alkyl als Rest aufweisen.

Sofern X,Y,Z unabhängig voneinander ein, zwei oder drei N bedeuten, gilt die Maßgabe, dass
1. das Skelett in der Teilgruppierung X-Y-Z nicht N-CH-N, CH-N-N oder N-N-N ist und
2. X nicht NH ist, wenn Y und Z gleichzeitig jeweils CH sind.

Bevorzugt gilt die Maßgabe, dass sofern X,Y,Z unabhängig voneinander ein, zwei oder drei N bedeuten
1. das Skelett in der Teilgruppierung X-Y=Z nicht N-N-CH, N-CH-N, CH-N-N oder N-N-N ist und
2. X nicht NH ist, wenn Y und Z gleichzeitig jeweils CH sind.

### Beispiel 1: Herstellung von 4-Methyl-3-(thieno[3,2-f]chinolin-9-ylarnino)-phenol

### Beispiel 1a) Herstellung von 2-(Benzo[b]thiophen-5-ylaminomethylen)-malonsäurediethylester

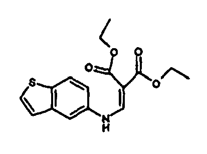

Eine Lösung von 540 mg Benzo[b]thiophen-5-ylamin in 5 ml Diethylethoxymethylenmalonat wird 1,5 Stunden lang bei 130 °C gerührt. Anschließend wird das Reaktionsgemisch mit Ethylacetat verdünnt. Man wäscht mit gesättigter wässriger Natriumchloridlösung, trocknet über Natriumsulfat und engt im Vakuum ein. Das Rohprodukt wird durch Säulenchromatographie an Kieselgel mit einem Gemisch aus Hexan/Ethylacetat gereinigt. Man erhält 1,88 g Produkt.
¹H-NMR (CDCl₃): δ= 1,30-1,45 (6H); 4,20-4,38 (4H); 7,16 (1H); 7,30 (1H); 7,51 (1H); 7,58 (1H); 7,86 (1H); 8,60 (1H) 11,12 (1H) ppm.

### Beispiel 1b) Herstellung von 9-Oxo-6,9-dihydro-thieno[3,2-f]chinolin-8-carbonsäure-ethylester

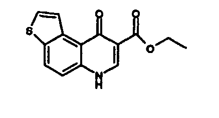

Eine Lösung von 315 mg der unter 1a beschriebenen Verbindung in 2 ml Diphenylether wird 35 Minuten lang bei 240 °C gerührt. Nach dem Abkühlen wird mit Cyclohexan versetzt und eine Stunde lang bei 23 °C nachgerührt. Das ausgefallene Produkt wird abgesaugt und aus einem Gemisch von Dichlormethan und Methanol (95:5) umkristallisiert. Man erhält 159 mg Produkt. ¹H-NMR (d6-DMSO): δ = 1,30 (3H); 4,23 (2H); 7,61 (1H); 8,02 (1H); 8,34 (1H); 8,56 (1H); 8,94 (1H); 12,50 (1H) ppm.

### Beispiel 1c) Herstellung von 9-Oxo-6,9-dihydro-thieno[3,2-f]chinolin-8-carbonsäure

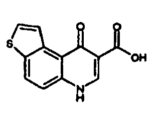

Zu einer Lösung von 1 g der unter Beispiel 1b beschriebenen Verbindung in 15 ml Ethanol wird eine Lösung von 500 mg Natriumhydroxid in Wasser addiert. Man kocht 2 Stunden lang unter Rückfluss. Nach dem Abkühlen wird mit 2 normaler Salzsäure angesäuert. Dann wird eine Stunde lang bei 23 °C nachgerührt. Anschließend wird abgesaugt. Man erhält 902 mg Produkt.
¹H-NMR (d6-DMSO): δ = 7,80 (1H); 8,18 (1H); 8,53 (1H); 8,84 (1H); 8,96 (1H); 13,67 (1H); 15,93 (1H) ppm.

### Beispiel 1d) Herstellung von 6H-Thieno[3,2-f]chinolin-9-on

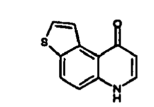

Eine Lösung von 100 mg der unter Beispiel 1c beschriebenen Verbindung in 3 ml Diphenylether wird bei 270 °C 1 Stunde lang gerührt. Nach dem Abkühlen wird mit Cyclohexan verdünnt und 8 Stunden lang bei 23 °C nachgerührt. Man filtriert und erhält 74 mg Produkt.
¹H-NMR (d6-DMSO): δ = 6,19 (1H); 7,55 (1H); 7,90-8,03 (2H); 8,26 (1H); 8,94 (1H); 11,99 (1H) ppm.

### Beispiel 1e) Herstellung von 9-Chlorthieno[3,2-f]chinolin

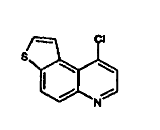

Eine Lösung von 150 mg der unter Beispiel 1 d beschriebenen Verbindung in 1,5 ml Thionylchlorid wird mit einem Tropfen N,N-Dimethylformamid versetzt und dann eine Stunde lang bei 100 °C gerührt. Anschließend wird das Reaktionsgemisch im Vakuum eingeengt. Es wird 3mal in Toluol gelöst und im Vakuum eingeengt. Dann wird das Produkt 20 Minuten lang mit 2 normaler Natronlauge gerührt. Man saugt ab, wäscht den Rückstand mit Wasser und trocknet im Vakuum bei 50 °C. Es werden 138 mg Produkt erhalten.
¹H-NMR (d6-DMSO): δ = 7,85 (1H); 8,00 (1H); 8,12 (1H); 8,46 (1H); 8,76-8,92 (2H) ppm.

### Beispiel 1f) Herstellung von 4-Methyl-3-(thieno[3,2-f]chinolin-9-ylamino)-phenol

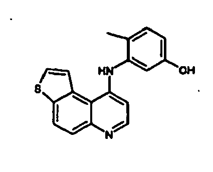

Ein Lösung von 130 mg der unter Beispiel 1e beschriebenen Verbindung sowie 85 mg 3-Hydroxy-6-methylanilin in 3 ml Acetonitril wird im Bombenrohr auf 160 °C erwärmt. Man belässt 24 Stunden lang bei dieser Temperatur, lässt dann abkühlen und engt das Reaktionsgemisch im Vakuum ein. Es wird an Kieselgel mit einem Gemisch aus Hexan/Ethylacetat chromatographiert. Man erhält 54 mg Produkt.
¹H-NMR (d6-DMSO): δ = 2,08 (3H); 6,47 (1 H); 6,50-6,61 (2H); 7,10 (1 H); 7,85 (1H); 7,96 (1 H); 8,10 (1 H); 8,29 (1 H); 8,40 (1 H); 8,54 (1H); 9,20 (1 H) ppm.

### Beispiel 2: Herstellung von 4-Methyl-3-(2-methyl-thiazolo[4,6-f]chinolin-9-ylamino)-phenol

### Beispiel 2a) Herstellung von 2-[(2-Methylbenzothiazol-5-ylamino)-methylen]-malonsäure-diethylester

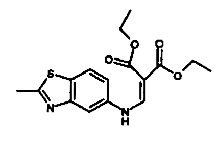

Analog zu Beispiel 1a werden aus 1 g 5-Amino-2-methylbenzothiazol in Diethylethoxymethylenmalonat 1,31 g Produkt erhalten.
¹H-NMR (CDCl₃): δ = 1,30-1,45 (6H); 2,84 (3H); 4,20-4,38 (4H); 7,15 (1H); 7,71 (1H); 7,78 (1H); 8,60 (1H); 11,13 (1H) ppm.

### Beispiel 2b) Herstellung von 2-Methyl-9-oxo-6,9-dihydrothiazolo[4,5-f]chinolin-8-carbonsäureethylester

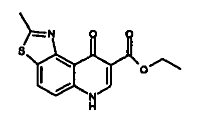

Analog zu Beispiel 1 b werden aus 1,31 g der unter 2a beschriebenen Verbindung in Diphenylether 1,09 g Produkt erhalten.
¹H-NMR (CDCl₃): δ = 1,46 (3H); 3,00 (3H); 4,50 (2H); 8,01 (1H); 8,13 (1H); 9,28 (1H); 13,11 (1H) ppm.

### Beispiel 2c) Herstellung von 2-Methyl-9-oxo-6,9-dihydro-thiazolo[4,5-f]chinolin-8-carbonsäure

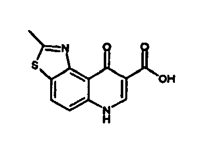

Analog zu Beispiel 1c werden aus 1,05 g der unter Beispiel 2b beschriebenen Verbindung 788 mg Produkt erhalten.
¹H-NMR (d6-DMSO): δ = 2,92 (3H); 7,80 (1 H); 8,53 (1 H); 8,91 (1 H); 13,55 (1 H) ppm.

### Beispiel 2d) Herstellung von 2-Methyl-6H-thiazolo[4,5-f]chinolin-9-on

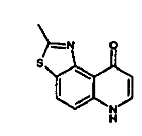

Analog zu Beispiel 1d werden aus 150 mg der unter Beispiel 2c) beschriebenen Verbindung in Diphenylether 55 mg Produkt erhalten.
¹H-NMR (d6-DMSO): δ = 2,92 (3H); 6,17 (1H); 7,61 (1H); 7,90 (1H); 8,28 (1H); 11,81 (1H) ppm.

### Beispiel 2e) Herstellung von 9-Chloro-2-methyl-thiazolo[4,5-f]chinolin

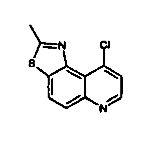

Analog zu Beispiel 1e werden aus 160 mg der unter Beispiel 2d beschriebenen Verbindung in Thionylchlorid 128 mg Produkt erhalten.
¹H-NMR (d6-DMSO): δ = 2,96 (3H); 7,92 (1H); 8,11 (1H); 8,56 (1H); 8,91 (1H) ppm.

### Beispiel 2f) Herstellung von 4-Methyl-3-(2-methyl-thiazolo[4,5-f]chinolin-9-yl-amino)-phenol

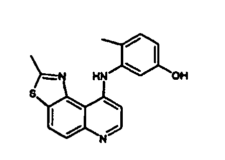

Analog zu Beispiel 1f werden aus 50 mg der unter Beispiel 2e beschriebenen Verbindung sowie 32 mg 3-Hydroxy-6-methylanilin in Acetonitril 41 mg Produkt erhalten.
¹H-NMR (d6-DMSO): δ = 2,24 (3H); 3,00 (3H); 6,60 (3H); 6,92 (1H); 6,99 (1H); 7,19 (1H); 7,88 (1H); 8,32 (1H); 8,51 (1H); 9,40 (1H); 10,95 (1H) ppm.

### Beispiel 3: Herstellung von 4-Methyl-3-(thieno[2,3-f]chinolin-9-ylamino)-phenol

### Beispiel 3a) Herstellung von 2-(Benzo[b]thiophen-6-ylaminomethylen)malonsäure-diethylester

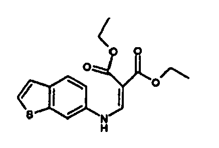

Analog zu Beispiel 1a werden aus 1 g 5-Amino-2-methylbenzothiazol in Diethylethoxymethylenmalonat 1,31 g Produkt erhalten.
¹H-NMR (d6-DMSO): δ= 1,20-1,35 (6H); 4,08-4,30 (4H); 7,43 (2H); 7,70 (1H); 7,89 (1H); 8,10 (1H); 8,50 (1H); 10,86 (1H) ppm.

### Beispiel 3b) Herstellung von 9-Oxo-6,9-dihydro-thieno[2,3-f]chinolin-8-carbonsäureethylester

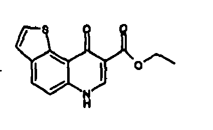

Analog zu Beispiel 1b werden aus 1,31 g der unter 3a beschriebenen Verbindung in Diphenylether 1,09 g Produkt erhalten.
¹H-NMR (d6-DMSO): δ = 1,33 (3H); 4,38 (2H); 7,62 (1H); 7,89 (1H); 8,24 (1H); 8,67 (1H); 12,76 (1H) ppm.

### Beispiel 3c) Herstellung von 9-Oxo-6,9-dihydro-thieno[2,3-f]chinolin-8-carbonsäure

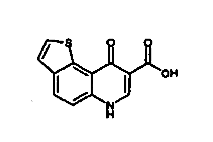

Analog zu Beispiel 2c werden aus 1,05 g der unter Beispiel 3b beschriebenen Verbindung 788 mg Produkt erhalten.
¹H-NMR (d6-DMSO): δ = 7,72 (1H); 7,84 (1H); 8,04 (1H); 8,41 (1H); 8,98 (1H); 13,78 (1H) ppm.

### Beispiel 3d) Herstellung von 6H-Thieno[2,3-f]chinolin-9-on

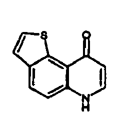

Analog zu Beispiel 1d werden aus 640 mg der unter Beispiel 3c beschriebenen Verbindung in Diphenylether 483 mg Produkt erhalten.
¹H-NMR (d6-DMSO): δ = 6,30 (1H); 7,55-7,66 (2H); 7,80 (1H); 8,03 (1H); 8,18 (1H); 12,23 (1H) ppm.

### Beispiel 3e) Herstellung von Trifluormethanesulfonsäure-thieno[2,3-f]chinolin-9-yl-ester

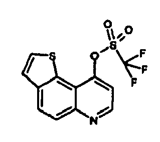

Zu einer Lösung von 100 mg der unter Beispiel 3d beschriebenen Substanz in 2 ml Pyridin werden bei 0 °C 250 µl Trifluormethansulfonsäureanhydrid addiert. Man lässt auf 21 °C kommen und rührt 45 Minuten lang bei dieser Temperatur nach. Anschließend wird das Reaktionsgemisch auf gesättigte wässrige Natriumchloridlösung gegossen. Man lässt 2 Stunden lang nachrühren und saugt dann ab. Der Rückstand wird durch Säulenchromatographie an Kieselgel mit einem Gemisch aus Hexan/Ethyacetat gereinigt. Man erhält 106 mg Produkt.
¹H-NMR (d6-DMSO): δ = 6,68 (1H); 7,68 (1H); 7,75 (1H); 7,94 (1H); 8,28-8,40 (1H) ppm.

### Beispiel 3f) Herstellung von 4-Methyl-3-(thieno[2,3-f]chinolin-9-ylamino)phenol

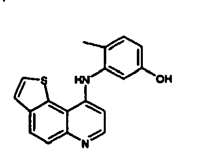

Eine Lösung von 100 mg der unter 3e beschriebenen Verbindung und 75 mg 3-Hydroxy-6-methylanilin in 5 ml Acetonitril wird 24 Stunden lang bei 50 °C gerührt. Anschließend wird das ausgefallene Reaktionsprodukt abgesaugt und durch Säulenchromatographie an Kieselgel mit einem Gemisch aus Hexan/Ethyacetat gereinigt. Man erhält 75 mg Produkt.
¹H-NMR (d6-DMSO): δ = 2,06 (3H); 6,60 (1 H); 6,78-6,90 (2H); 7,26 (1 H); 7,91 (1H); 8,08 (1H); 8,21 (1H); 8,52-8,65 (2H); 9,38 (1H) ppm.

### Beispiel 4: Herstellung von 3-(3,3-Dioxo-2,3-dihydro-1H-3λ⁶-thieno[3,2-f]chinolin-9-ylamino)-4-methyl-phenol

### Beispiel 4a) Herstellung von 2-[(1,1-Dioxo-2,3-dihydro-1H-1λ⁶-benzo[b]thiophen-5-ylamino)-methylen]malonsäurediethylester

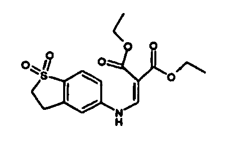

Analog zu Beispiel 1a werden aus 340 mg 1,1-Dioxo-2,3-dihydro-1H-1λ⁶ -benzo[b]thiophen-5-ylamin in Diethylethoxymethylenmalonat 613 mg Produkt erhalten.
¹H-NMR (d6-DMSO): δ = 1,25 (6H); 3,32 (2H); 3,59 (2H); 4,18 (2H); 7,50 (1H); 7,54 (1H); 7,72 (1H); 8,42 (1H); 10,72 (1H) ppm.

### Beispiel 4b) Herstellung von 3,3,9-Trioxo-2,3,6,9-tetrahydro-1H-3λ⁶-thieno[3,2-f]chinolin-8-carbonsäureethylester

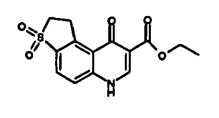

Analog zu Beispiel 1 b werden aus 100 mg der unter 4a beschriebenen Verbindung in Diphenylether 162 mg Produkt erhalten.
¹H-NMR (d6-DMSO): δ = 1,28 (3H); 3,62 (2H); 3,96 (2H); 4,22 (2H); 7,72 (1H); 7,96 (1H); 8,56 (1H); 12,60 (1H) ppm.

### Beispiel 4c) Herstellung von 3,3,9-Trioxo-2,3,6,9-tetrahydro-1H-3λ⁶-thieno-[3,2-f]chinolin-8-carbonsäure

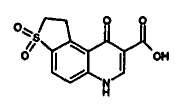

Analog zu Beispiel 1 c werden aus 444 mg der unter Beispiel 4b beschriebenen Verbindung 382 mg Produkt erhalten.
¹H-NMR (d6-DMSO): δ = 3,69 (2H); 4,00 (2H); 7,90 (1H); 8,12 (1H); 8,97 (1H); 13,66 (1H); 14,98 (1H) ppm.

### Beispiel 4d) Herstellung von 3,3-Dioxo-1,2,3,6-tetrahydro-3λ⁶-thieno[3,2-f]chinolin-9-on

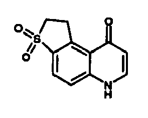

Analog zu Beispiel 1d werden aus 380 mg der unter Beispiel 4c beschriebenen Verbindung in Diphenylether 280 mg Produkt erhalten.
¹H-NMR (d6-DMSO): δ = 3,59 (2H); 3,95 (2H); 6,11 (1H); 7,63 (1H); 7,85-8,02 (2H); 12,09 (1H) ppm.

### Beispiel 4e) Herstellung von 9-Chloro-1,2-dihydro-thieno[3,2-f]chinolin 3,3-dioxid

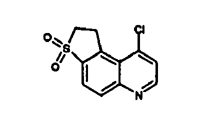

Analog zu Beispiel 1e werden aus 500 mg der unter Beispiel 4d beschriebenen Verbindung in Thionylchlorid 512 mg Produkt erhalten.
¹H-NMR (d6-DMSO): δ = 3,77 (2H); 4,16 (2H); 7,90 (1 H); 8,06 (1 H); 8,21 (1 H); 8,95 (1 H) ppm.

### Beispiel 4f) Herstellung von 3-(3,3-Dioxo-2,3-dihydro-1H-3λ⁶-thieno[3,2-f]chinolin-9-ylamino)-4-methyl-phenol

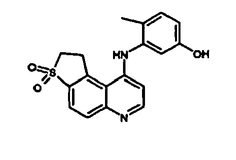

Analog zu Beispiel 1f werden aus 83 mg der unter Beispiel 4e beschriebenen Verbindung sowie 80 mg 3-Hydroxy-6-methylanilin in Acetonitril 51 mg Produkt erhalten.
¹H-NMR (d6-DMSO): δ = 2,10 (3H); 3,80 (2H); 4,24 (2H); 6,49 (1H); 6,79 (1H); 6,84 (1H); 7,26 (1H); 8,19 (1 H); 8,28 (1H); 8,52 (1 H); 9,61 (1H); 9,89 (1H) ppm.

### Beispiel 5: Herstellung von 3-(3,3-Dioxo-2,3-dihydro-1H-3λ⁶-thleno[3,2-f]quinolin-9-ylamino)-phenol

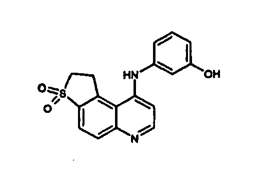

Analog zu Beispiel 4f werden aus 90 mg der unter Beispiel 4e beschriebenen Verbindung sowie 80 mg 3-Aminophenol in Acetonitril 73 mg Produkt erhalten.
¹H-NMR (d6-DMSO): δ = 3,76 (2H); 4,22 (2H); 6,82 (1H); 6,90 (2H); 7,02 (1H); 8,18 (1H); 8,28 (1H); 8,58 (1H); 9,80 (1H); 9,98 (1H) ppm.

### Beispiel 6: Herstellung von 4-(3,3-Dioxo-2,3-dihydro-1H-3λ⁶-thieno[3,2-f]chinolin-9-ylamino)-3-methyl-phenol

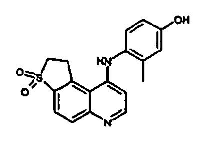

Analog zu Beispiel 4f werden aus 90 mg der unter Beispiel 4e beschriebenen Verbindung sowie 90 mg 4-Amino-3-methyl-phenol in Acetonitril 37 mg Produkt erhalten.
¹H-NMR (d6-DMSO): δ = 3,78 (2H); 4,26 (2H); 6,34 (1H); 6,80 (1H); 6,88 (1H); 7,12 (1H); 8,15 (1H); 8,28 (1H); 8,48 (1H); 9,43 (1H); 9,84 (1H) ppm.

### Beispiel 7: Herstellung von 2-(3,3-Dioxo-2,3-dihydro-1H-3λ⁶-thieno[3,2-f]chinolin-9-ylamino)-phenol

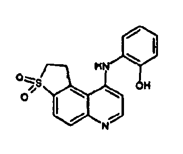

Analog zu Beispiel 4f werden aus 90 mg der unter Beispiel 4e beschriebenen Verbindung sowie 80 mg 2-Aminophenol in Acetonitril 59 mg Produkt erhalten.
¹H-NMR (d6-DMSO): δ = 3,80 (2H); 4,22 (2H); 6,52 (1H); 6,99 (1H); 7,12 (1H); 7,28-7,40 (2H); 8,16 (1H); 8,28 (1H); 8,54 (1H); 9,48 (1H); 10,20 (1H) ppm.

### Beispiel 8: Herstellung von 4-(3,3-Dioxo.2,3-dihydro-1H-3λ⁶-thieno[3,2-f]quinolin-9-ylamino)-phenol

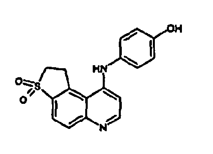

Analog zu Beispiel 4f werden aus 90 mg der unter Beispiel 4e beschriebenen Verbindung sowie 80 mg 4-Aminophenol in Acetonitril 47 mg Produkt erhalten.
¹H-NMR (d6-DMSO): δ = 3,74 (2H); 4,22 (2H); 6,78 (1H); 6,95 (2H); 7,28 (2H); 8,12 (1H); 8,24 (1H); 8,50 (1H); 9,65 (1H); 9,91 (1H) ppm.

### Beispiel 9: Herstellung von [3-(3,3-Dioxo-2,3-dihydro-1H-3λ⁶-thieno[3,2-f]quinolin-9-ylamino)phenyl]-methanol

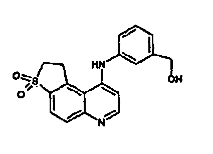

Analog zu Beispiel 4f werden aus 90 mg der unter Beispiel 4e beschriebenen Verbindung sowie 90 mg 3-Aminobenzylalkohol in Acetonitril 88 mg Produkt erhalten.
¹H-NMR (d6-DMSO): δ = 3,76 (2H); 4,25 (2H); 4,58 (2H); 7,00 (1H); 7,35 (2H); 7,46 (1H); 7,53 (1H); 8,18 (1H); 8,39 (1H); 8,59 (1H); 9,99 (1H) ppm.

### Beispiel 10: Herstellung von 3-(3,3-Dioxo-2,3-dihydro-1H-3λ⁶-thieno[3,2-f]chinolin-9-ylamino)-benzoesäure

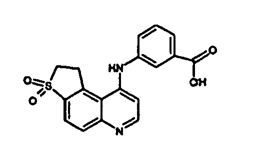

Analog zu Beispiel 4f werden aus 90 mg der unter Beispiel 4e beschriebenen Verbindung sowie 100 mg 3-Aminobenzoesäure in Acetonitril 65 mg Produkt erhalten.
¹H-NMR (d6-DMSO): δ = 3,73(2H); 4,28 (2H); 7,06 (1H); 7,66-7,83 (2H); 7,95 (1H); 8,06 (1H); 8,20 (1H); 8,30 (1H); 8,61 (1H); 10,00 (1H); 13,28 (1H) ppm.

### Beispiel 11: Herstellung von 3-(3,3-Dioxo-2,3-dihydro-1H-3λ⁶-thieno[3,2-f]chinolin-9-ylamino)-benzamid

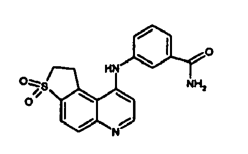

Analog zu Beispiel 4f werden aus 90 mg der unter Beispiel 4e beschriebenen Verbindung sowie 100 mg 3-Aminobenzamidin Acetonitril 54 mg Produkt erhalten.
¹H-NMR (d6-DMSO): δ = 3,78 (2H); 4,28 (2H); 7,03 (1H); 7,52 (1H); 7,67 (2H); 7,90 (1H); 7,99 (1H); 8,12 (1H); 8,21 (1H); 8,29 (1H); 8,60 (1H); 9,98 (1H) ppm.

### Beispiel 12: Herstellung von (3,3-Dioxo-2,3-dihydro-1H-3λ⁶-thieno[3,2-f]quinolin-9-yl)-(3-methoxyphenyl)amin

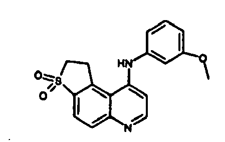

Analog zu Beispiel 4f werden aus 90 mg der unter Beispiel 4e beschriebenen Verbindung sowie 100 mg 3-Methoxyphenylamin in Acetonitril 106 mg Produkt erhalten.
¹H-NMR (d6-DMSO): δ = 3,70-3,88 (5H); 4,26 (2H); 6,95-7,16 (4H); 7,50 (1H); 8,19 (1H); 8,28 (1H); 8,58 (1H); 9,90 (1H) ppm.

### Beispiel 13: Herstellung von N-[3-(3,3-Dioxo-2,3-dihydro-1H-3λ⁶-thieno[3,2-f]chinolin-9-ylamino)-phenyl]-acetamid

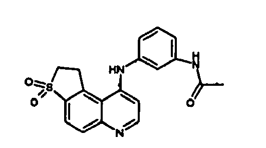

Analog zu Beispiel 4f werden aus 150 mg der unter Beispiel 4e beschriebenen Verbindung sowie 180 mg N-(3-Aminophenyl)-acetamid in Acetonitril 146 mg Produkt erhalten.
¹H-NMR (d6-DMSO): δ = 2,08 (3H); 3,74 (2H); 4,23 (2H); 7,02 (1H); 7,16 (1H); 7,40-7,55 (2H); 7,96 (1H); 8,18 (1H); 8,28 (1H); 8,59 (1H); 9,90 (1H); 10,32 (1H) ppm.

### Beispiel 14: Herstellung von 3-(3,3-Dioxo-2,3-dihydro-1H-3λ⁶-thieno[3,2-f]chinolin-9-ylamino)-5-methoxyphenol

### Beispiel14a)Herstellung von Trifluoromethanesulfonsäure-3,3-dioxo-2,3-dihydro-1H-3λ⁶-thieno[3,2-f]chinolin-9-yl-ester

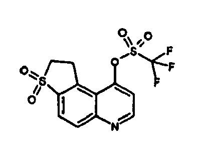

Analog zu Beispiel 3e werden aus 300 mg der unter Beispiel 4d beschriebenen Verbindung und 645 µl Trifluormethansulfonsäureanhydrid in Pyridin 348 mg Produkt erhalten.
¹H-NMR (d6-DMSO): δ = 3,78 (2H); 3,90 (2H); 7,86 (1H); 8,17 (1H); 8,30 (1H); 9,20 (1 H) ppm.

### Beispiel 14b) Herstellung von 3-(3,3-Dioxo-2,3-dihydro-1H-3λ⁶-thieno[3,2-f]chinolin-9-ylamino)-5-methoxyphenol

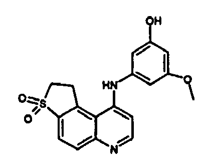

Analog zu Beispiel 3f werden aus 100 mg der unter 14a beschriebenen Verbindung und 85 mg 3-Amino-5-methoxyphenol in Acetonitril 59 mg Produkt erhalten.
¹H-NMR (d6-DMSO): δ = 3,60-3,75 (5H); 4,07 (2H); 6,06 (1H); 6,28 (2H); 7,35 (1 H); 7,89 (1 H); 8,00 (1 H); 8,27 (1 H); 8,66 (1 H); 9,50 (1 H) ppm.

### Beispiel 15: Herstellung von 5-(3,3-Dioxo-2,3-dihydro-1H-3λ⁶-thieno[3,2-f]chinolin-9-ylamino)-2-methylphenol

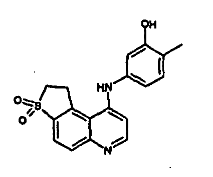

Analog zu Beispiel 14b werden aus 120 mg der unter Beispiel 14a beschriebenen Verbindung und 80 mg 5-Amino-2-methylphenol in Acetonitril 58 mg Produkt erhalten.
¹H-NMR (d6-DMSO): δ = 2,10 (3H); 3,67 (2H); 4,10 (2H); 6,62 (1H); 6,74 (1H); 7,06 (1H); 7,17 (1H); 7,87 (1H); 7,98 (1H); 8,18 (1H); 8,59 (1H); 9,40 (1H) ppm.

### Beispiel 16: Herstellung von 3-(3,3-Dioxo-2,3-dihydro-1H-3λ⁶-thieno[3,2-f]chinolin-9-ylamino)-2-methylphenol

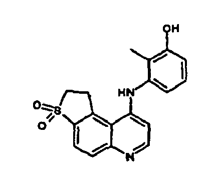

Analog zu Beispiel 14b werden aus 120 mg der unter Beispiel 14a beschriebenen Verbindung und 80 mg 3-Amino-2-methylphenol in Acetonitril 32 mg Produkt erhalten.
¹H-NMR (d6-DMSO): δ = 2,00 (3H); 3,75 (2H); 4,27 (2H); 6,35 (1H); 6,71 (1H); 7,00 (1H): 7,18 (1H); 8,48 (1H) ppm.

In Analogie zu dem unter Beispiel 14b beschriebenen Verfahren werden aus der unter 14a beschriebenen Verbindung und dem jeweiligen Anilinderivat die in der folgenden Tabelle gezeigten Beispiele hergestellt:

| Beispiel | Struktur | Name | δ 1H-NMR ppm |
|---|---|---|---|
| 17 | 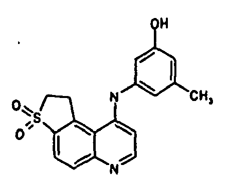 | 3-(3,3-Dioxo-2,3-dihydro-1H-3λ⁶-thieno[3,2-f]chinolin-9-ylamino)-5-methyl-phenol | (d6-DMSO, 400 MHz): 2,16 (3H); 3,61 (2H); 4,03 (2H); 6,28 (1H); 6,47 (2H); 7,23 (1H); 7,84 (1H); 7,93 (1H); 8,18 (1H); 8,60 (1H); 9,33 (1H) |
| 18 | 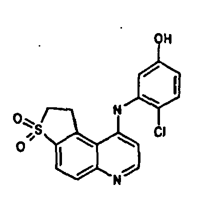 | 4-Chloro 3-(3,3-dioxo-2,3-dihydro-1H-3λ⁶ -thieno[3,2-f]chinolin-9-ylamino)-5-methyl-phenol | (d6-DMSO, 1 Tropfen DCI; 300 MHz): 3,74 (2H); 4,22 (2H); 6,48 (1H); 6,91 (1H); 7,02 (1H); 7,46 (1H); 8,25 (2H); 8,57 (1H) |
| 19 | 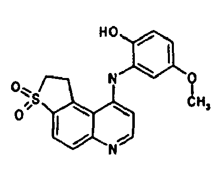 | 2-(3,3-Dioxo-2,3-dihydro-1H-3λ⁶-thieno[3,2-f]chinolin-9-ylamino)-4-methoxy-phenol | (d6-DMSO, 1 Tropfen DCI; 300 MHz): 3,67 (3H); 3,71 (2H); 4,23 (2H); 6,52 (1H); 6,86 (1H); 6,97-7,06 (2H); 8,21 (2H); 8,50 (1 H) |
| 20 | 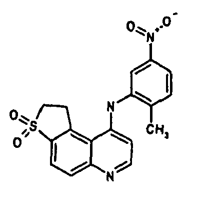 | (3,3-Dioxo-2,3-dihydro-1H-3λ⁶-thieno[3,2-f]-chinolin-9-yl)-(2-methyl-5-nitro-phenyl)-amin | (d6-DMSO, 1 Tropfen DCI; 300 MHz): 2,29 (3H); 3,72 (2H); 4,33 (2H); 6,41 (1H); 7,71 (1H); 8,15-8,37 (4H); 8,52 (1 H) |
| 21 | 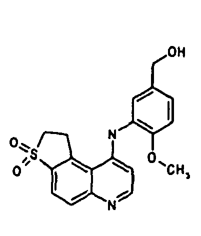 | [3-(3,3-Dioxo-2,3-dihydro-1H-3λ⁶-thieno[3,2-f]quinolin-9-ylamino)-4-methoxy-phenyl]-methanol | (d6-DMSO, 300 MHz): 3,71-3,82 (5H); 4,16 (2H); 4,47 (2H); 6,51 (1 H); 7,21 (1 H); 7,35 (2H); 8,01 (1H); 8,22 (1 H); 8,50 (1 H); 9,40 (1H) |
| 22 | 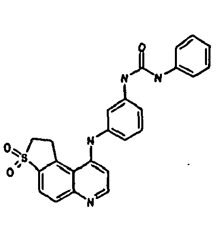 | 1-[3-(3,3-Dioxo-2,3-dihydro-1H-3λ⁶-thieno[3,2-]chinolin-9-ylamino)-phenyl]-3-phenyl-harnstoff | (d6-DMSO, 400 MHz): 3,77 (2H); 4,21 (2H); 6,98 (1H); 7,08 (2H); 7,29 (3H); 7,42-7,52 (3H); 7,87 (1H); 8,05 (1H); 8,28 (1H); 8,61 (1H); 8,74 (1H); 8,95 (1H); 9,76 (1H) |
| 23 | 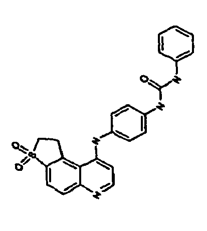 | 1-[4-(3,3-Dioxo-2,3-dihydro-1H-3λ⁶-thieno[3,2-f]quinolin-9-ylamino)-phenyl]-3-phenyl-harnstoff | (d6-DMSO, 400 MHz): 3,72 (2H); 4,17 (2H); 6,86 (1H); 6,95 (1H); 7,25 (2H); 7,35 (2H); 7,43 (2H); 7,63 (2H); 7,99 81H); 8,22 (1H); 8,50 (1H); 8,70 (1H); 8,90 (1H); 9,61 (1H) |
| 24 | 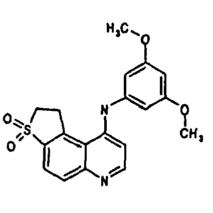 | (3,5-Dimethoxy-phenyl)-(3,3-dioxo-2,3-dihydro-1H-3λ⁶-thieno[3,2-f]chinolin-9-yl)-amin | (d6-DMSO, 300 MHz): 3,68-3,84 (8H); 4,16 (2H); 6,52 (1H); 6,60 (2H); 7,10 (1H);8,01 (1H); 8,23 (1H); 8,56 (1H); 9,60 (1H) |
| 25 | 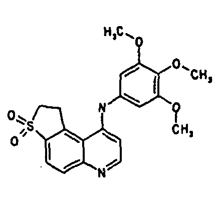 | (3,3-Dioxo-2,3-dihydro-1H-3λ⁶-thieno[3,2-f]-chinolin-9-yl)-(3,4, 5-trimethoxy-phenyl)-amin | (d6-DMSO, 400 MHz): 3,66-3,82 (11H); 4,17 (2H); 6,78 (2H); 7,05 (1H); 8,00 (1H); 8,21 (1H); 8,51 (1H); 9,57 (1H) |

Die zur Herstellung der Beispiele 22 und 23 benötigten Aniline werden wie folgt dargestellt:

### 1-(3-Amino-phenyl)-3-phenyl-harnstoff (für Beispiel 22)

3 g 3-Nitroanilin werden in 50 ml Dichlormethan gelöst. Man addiert 3,5 ml Phenylisocyanat und lässt 22 Stunden bei 23°C nachrühren. Anschließend wird das ausgefallene Reaktionsprodukt abfiltriert. Man löst das Rohprodukt in einem Gemisch aus 30 ml Tetrahydrofuran und 16 ml Ethanol, addiert 150 mg Palladium/Kohle (10%) setzt unter Wasserstoff und hydriert bei Normaldruck für 1,5 Stunden. Dann wird das Reaktionsgemisch über Celite filtriert. Man engt im Vakuum ein und kristallisiert das erhaltene Rohprodukt aus Diisopropylether um. Es werden 2,3 g Produkt erhalten.

### 1-(4-Amino-phenyl)-3-phenyl-harnstoff (für Beispiel 23)

Die Herstellung erfolgt analog zum oben genannten Verfahren für 1-(3-Aminophenyl)-3-phenyl-harnstoff, wobei 4-Nitroanilin als Augangsmaterial verwendet wird.

### Beispiel 26

### Herstellung von N³-(3,3-Dioxo-2,3-dihydro-1H-3λ⁶-thieno[3,2-f]quinolin-9-yl)-4-methyl-phenyl-1,3-diamin

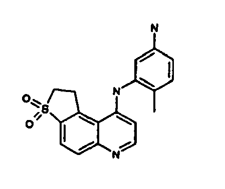

100 mg der unter Beispiel 20 beschriebenen Verbindung werden in einem Gemisch aus 5 ml Tetrahydrofuran und 3 ml Ethanol gelöst. Man addiert 20 mg Palladium/Kohle (10%) setzt unter Wasserstoff und hydriert bei Normaldruck für 4,5 Stunden. Dann wird das Reaktionsgemisch über Celite filtriert. Man engt im Vakuum ein und reinigt das erhaltene Rohprodukt durch Säulenchromatographie an Kieselgel. Es werden 91 mg Produkt erhalten.
(d6-DMSO, 1 Tropfen DCI; 400 MHz): δ = 2,18 (3H); 3,70 (2H); 4,27 (2H); 6,31 (1 H); 7,41 (1 H); 7,46 (1 H); 7,54 (1 H); 8,23 (2H); 8,50 (1 H) ppm.

### Beispiel 27

### Herstellung von 1-[3-(3,3-Dioxo-2,3-dihydro-1H-3λ⁶-thieno[3,2-f]chinolin-9-ylamino)-4-methyl-phenyl]-3-phenyl-harnstoff

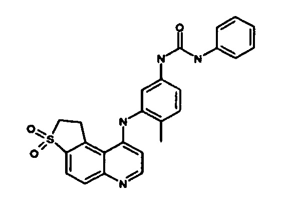

22 mg der unter Beispiel 26 beschriebenen Verbindung werden in 2 ml Dichlormethan gelöst. Man addiert 12 µl Phenylisocyanat und lässt 16 Stunden bei 23°C nachrühren. Danach wird mit etwas Diisopropylether verdünnt. Man filtriert das ausgefahene Reaktionsprodukt ab und rührt dann das Rohprodukt mit Disopropylether aus. Es werden 20 mg Produkt erhalten.
(d6-DMSO, 1 Tropfen DCI; 400 MHz): δ = 2,18 (3H); 3,12 (2H); 4,22 (2H); 6,34 (1H); 6,87 (1H); 7,18 (2H); 7,30 (1H); 7,36 (3H); 7,54 (1H); 8,20 (2H); 8,46 (1H) ppm.

### Biologische Tests der Verbindungen

### Testsystem für EphB4

Eine Mischung aus 20 ng/ml rekombinanter EphB4 kinase (ProQinase GmbH, Freiburg, Germany), 2.67 µg/ml polyGluAlaTyr, 2 µM ATP, 25 mM HEPES (pH 7.3), 5 mM MgCl₂, 1 mM MnCl₂, 2 mM DTT, 0.1 mM NaVO₄, 1% (v/v) Glycerin, 0.02% NP40, EDTA-freie Protease Inhibitoren (Complete Fa. Roche, 1 Tablette in 50 ml) wird bei 20 °C 10 min lang inkubiert. Testsubstanzen werden in 100% DMSO gelöst und in 0.017-fachem Volumen vor dem Start der Reaktion vorgelegt. 60 Minuten nach Zugabe von 1.7-fachem Volumen einer Lösung 50mM Hepes pH 7.0, 0.2 % BSA, 0.14 µg/ml PT66-Europium, 3.84 µg/ml SA-XL665, 75 mM EDTA wird der Ansatz in einem Discovery HTRF-Messgerät der Firma PerkinElmer vermessen.

Unter anderen hemmen folgende Verbindungen die EphB4 Kinase mit einer IC₅₀, die kleiner als 25 µM ist: Erfindungsgemäße Beispiele 1, 2, 3, 4, 5 und 15 der Beschreibung. Die IC₅₀ der Verbindung nach Beispiel 2 beträgt 270nM.

Dies verdeutlicht, dass die erfindungsgemäßen Substanzen Rezeptortyrosinkinasen, insbesondere Eph Rezeptoren und hier insbesondere EphB4 inhibieren.

## Patentansprüche

1. Chinolinderivat mit der allgemeinen Formel **A**: 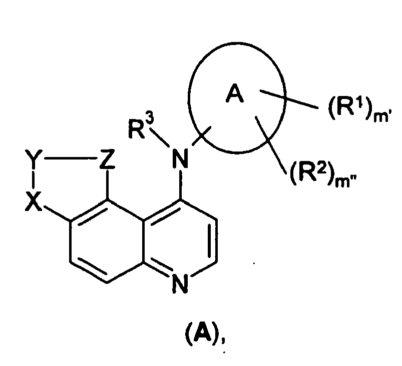 wobei
A ausgewählt ist aus der Gruppe, umfassend -C₆-C₁₂-Aryl, -C₅-C₁₈- Heteroaryl, -C₃-C₁₂Cycloalkyl und -C₃-C₁₂-Heterocycloalkyl,
R¹ und R² gleich oder verschieden und einfach oder mehrfach unabhängig voneinander ausgewählt sind aus der Gruppe, umfassend Was- serstoff, Hydroxy, Halogen, Nitro, Cyano, -C₁-C₆-Alkyl, -C₁-C₄- Hydroxyalkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, -C₁-C₆- Alkoxy, -C₁-C₆-Alkoxy-C₁-C₆-alkoxy, -C₁-C₆-Alkoxy-C₁-C₆-alkyl, -C₁-C₆-Alkoxy-C₁-C₆-alkoxy-C₁-C₆-alkyl, -(CH₂)ₙ-C₆-C₁₂-Aryl, -(CH₂)ₙ-C₅-C₁₈-Heteroaryl, -(CH₂)ₙ-C₃-C₁₀-Cycloalkyl, -(CH₂)ₙ-C₃- C₁₂-Heterocycloalkyl, -Phenylen-(CH₂)ₚ-R⁶, -(CH₂)ₚPO₃(R⁶)₂, -(CH₂)ₚ-NR⁵R⁶, -(CH₂)ₚ-NR⁴COR⁵, -(CH₂)ₚ-NR⁴CSR⁵, -(CH₂)ₚ- NR⁴S(O)R⁵, -(CH₂)ₚ-NR⁴S(O)₂R⁵, -(CH₂)ₚ-NR⁴CONR⁵R⁶, -(CH₂)ₚ-NR⁴COOR⁵, -(CH₂)ₚ-NR⁴C(NH)NR⁵R⁶, -(CH₂)ₚ-NR⁴CSNR⁵R⁶, -(CH₂)ₚ-NR⁴S(O)NR⁵R⁶, -(CH₂)ₚ- NR⁴S(O)₂NR⁵R⁶, -(CH₂)ₚ-COR⁵, -(CH₂)ₚ-CSR⁵, -(CH₂)ₚ-S(O)R⁵, -(CH₂)ₚ-S(O)(NH)R⁵, -(CH₂)ₚ-S(O)₂R⁵, -(CH₂)ₚ-S(O)₂NR⁵R⁶, - (CH₂)ₚ-SO₂OR⁵, -(CH₂)ₚ-CO₂R⁵, -(CH₂)ₚ-CONR⁵R⁶, -(CH₂)ₚ- CSNR⁵R⁶, -OR⁵, -(CH₂)ₚ-SR⁵ und -CR⁵(OH)-R⁶, wobei -C₁-C₆- Alkyl, -C₂-C₆-Alkenyl, -C₂-C₆-Alkinyl, -C₃-C₁₀-Cycloalkyl, -C₃-C₁₂- Heterocycloalkyl, -C₆-C₁₂-Aryl, -C₅-C₁₈-Heteroaryl und/oder -C₁- C₆-Alkoxy unsubstituiert sind oder einfach oder mehrfach unab- hängig voneinander mit Hydroxy, Halogen, Nitro, Cyano, Phenyl, - NR⁵R⁶, Alkyl und/oder -OR⁵ substituiert sind, wobei das Kohlen- stoffgerüst des -C₃-C₁₀-Cycloalkyls und des -C₁-C₁₀-Alkyis einfach oder mehrfach unabhängig voneinander Stickstoff-, Sauerstoff-, Schwefelatome und/oder C=O-Gruppen und/oder eine oder meh- rere Doppelbindungen enthalten kann, und/oder R¹ und R² optio- nal miteinander eine Brücke aus 3-10 Methyleneinheiten bilden, wobei bis zu zwei Methyleneinheiten mit O S und/oder -NR⁴ opti- onal ersetzt werden,
X, Y, Z gleich oder verschieden und unabhängig voneinander ausgewählt sind aus der Gruppe, umfassend -CR³=, -CR³R⁴-, -C(O)-, -N=, -S-, -O-, -NR³-, -S(O)₂-, -S(O)- und -S(O)NH-, und sich zwischen X, Y und Z Einfach- oder Doppelbindungen befinden,
R³ Wasserstoff, -C₁-C₁₀-Alkyl oder -C₁-C₁₀-Alkanoyl ist,
R⁴ Wasserstoff oder -C₁-C₁₀-Alkyl,
R⁵ und R⁶ gleich oder verschieden und unabhängig voneinander ausgewählt sind aus der Gruppe, umfassend Wasserstoff, -C₁-C₁₀-Alkyl, -C₂- C₁₀-Alkenyl, -C₂-C₁₀-Alkinyl, -C₁-C₆-Alkoxy, -C₃-C₁₀-Cycloalkyl, - C₃-C₁₂-Heterocycloalkyl, -C₆-C₁₂-Aryl und -C₅-C₁₈-Heteroaryl, wo- bei -C₁-C₁₀-Alkyl, -C₂-C₁₀-Alkenyl, -C₂-C₁₀-Alkinyl, -C₁-C₆-Alkoxy, - C₃-C₁₀-Cycloalkyl, -C₃-C₁₂-Heterocycloalkyl, -C₆-C₁₂-Aryl und/oder -C₅-C₁₈-Heteroaryl unsubstituiert sind oder einfach oder mehrfach unabhängig voneinander mit Hydroxy, Halogen, Cyano, Nitro, -OR⁷, -NR⁷R⁸, -C(O)NR⁷R⁸, -C(O)OR⁷ und/oder -C₁-C₆-Alkyl, wo- bei -C₁-C₆-Alkyl unsubstituiert ist oder einfach oder mehrfach un- abhängig voneinander mit Halogen, Hydroxy, Cyano, -NR⁷R⁸, -OR⁷ und/oder Phenyl; und/oder R⁵ und R⁶ optional miteinander eine Brücke aus 3-10 Methyleneinheiten bilden, wobei bis zu zwei Methyleneinheiten mit O, S und/oder NR⁴ optional ersetzt werden;
R⁷, R⁸ gleich oder verschieden und unabhängig voneinander ausgewählt sind aus der Gruppe, umfassend Wasserstoff, -C₁-C₄-Alkyl, -C₆- C₁₂-Aryl and -C₅-C₁₈-Heteroaryl, wobei Alkyl, Aryl, Heteroaryl un- substituiert ist oder einfach oder mehrfach unabhängig voneinan- der mit Halogen und/oder Alkoxy, oder R⁷ und R⁸ optional mitein- ander eine Brücke aus 3-10 Methyleneinheiten bilden, wobei bis zu zwei Methyleneinheiten mit O, S und/oder -NR⁴ optional ersetzt werden;
m', m" = unabhängig voneinander 0 - 4,
n =1-6,
p = 0 - 6, sowie
deren N-Oxide, Solvate, Hydrate, Stereoisomere, Diastereomere, Enantiomere und Salze
mit der Maßgabe, dass wenn X,Y,Z unabhängig voneinander ein, zwei oder drei N bedeuten,
1. das Skelett in der Teilgruppierung X-Y-Z nicht
N-CH-N, CH-N-N oder N-N-N ist und
2. X nicht NH ist, wenn Y und Z gleichzeitig jeweils CH sind.

2. Chinolinderivat nach Anspruch 1, **dadurch gekennzeichnet, dass** wenn X,Y,Z unabhängig voneinander ein, zwei oder drei N bedeuten,
1. das Skelett in der Teilgruppierung X-Y-Z nicht N-N-CH,
N-CH-N, CH-N-N oder N-N-N ist und
2. X nicht NH ist, wenn Y und Z gleichzeitig jeweils CH sind.

3. Chinolinderivat nach Anspruch 1 und/oder 2, **dadurch gekennzeichnet, dass** A Phenyl ist.

4. Chinolinderivat nach Anspruch 3, **dadurch gekennzeichnet, dass**
R¹ und R² gleich oder verschieden und einfach oder mehrfach unabhängig voneinander ausgewählt sind aus der Gruppe, umfassend Was- serstoff, Hydroxy, Halogen, Nitro, Cyano, -C₁-C₆-Alkyl, -C₁-C₄- Hydroxyalkyl, -C₆-C₁₂-Aryl, -C₁-C₆-Alkoxy, -NR⁵R⁶, -NR⁴COR⁵, -NR⁴S(O)R⁵, -NR⁴S(O)₂R⁵, -NR⁴CONR⁵R⁶, -NR⁴S(O)NR⁵R⁶, -NR⁴S(O)₂NR⁵R⁶, -COR⁵, COOR⁵, -S(O)R⁵, -S(O)(NH)R⁵, - S(O)₂R⁵, -S(O)₂NR⁵R⁶, -CO₂R⁵, -CONR⁵R⁶, -OR⁵ und -CR⁵(OH)- R⁶, und
m', m" = unabhängig voneinander 0 - 3.

5. Chinolinderivat nach einem der vorhergehenden Ansprüche, **dadurch gekennzeichnet, dass** X, Y und Z unabhängig voneinander ausgewählt sind aus der Gruppe, umfassend -CR⁴=, -CR⁴R⁵-, -C(O)-, -N=, -S-, -O-, -NR⁴-, -S(O)₂-, -S(O)- und -S(O)NH-, wobei N, S oder O im Ring nicht mehrfach vorkommen.

6. Chinolinderivat nach einem der vorhergehenden Ansprüche, **dadurch gekennzeichnet, dass** X, Y und Z für -S(O)₂-, -S-, -NH-, -CH=, -C(CH₃)=, und/oder -CH₂- stehen.

7. Chinolinderivat nach einem der vorhergehenden Ansprüche, **dadurch gekennzeichnet, dass** das Skelett in der Teilgruppierung X-Y-Z ausgewählt ist aus der Gruppe, umfassend -S-CH=CH-, -S-C(C₁-C₆-Alkyl)=N-, -S(O)₂-CH₂-CH₂- und -CH=CH-S-.

8. Chinolinderivat nach einem der vorhergehenden Ansprüche, **dadurch gekennzeichnet, dass** R³ Wasserstoff ist.

9. Chinolinderivat nach einem der vorhergehenden Ansprüche, **dadurch gekennzeichnet, dass**
A Phenyl,
R¹ und R² gleich oder verschieden und einfach oder mehrfach unabhängig voneinander ausgewählt sind aus der Gruppe, umfassend Was- serstoff, Hydroxy, Halogen, Nitro, Amino, Cyano, -C₁-C₆-Alkyl, - C₁-C₄-Hydroxyalkyl, -C₁-C₆-Alkoxy, -C₁-C₄-Alkyl-CO-NH-, -NH- C(O)-NH-Aryl, -COOR⁵, -CR⁵(OH)-R⁶ und -CONH₂, und
m', m" unabhängig voneinander 0 - 3.

10. Chinolinderivat nach Anspruch 9, **dadurch gekennzeichnet, dass**
R¹ und R² gleich oder verschieden und einfach oder mehrfach unabhängig voneinander ausgewählt sind aus der Gruppe, umfassend Was- serstoff, Hydroxy, Halogen, Nitro, Amino, Cyano, -CH₃, -C₂H₅, CH₃O-, C₂H₅O-, HOCH₂-, CH₃CONH-, -NH-C(O)-NH-Phenyl, - COOH und -CONH₂.

11. Verwendung des Chinolinderivats nach einem der Ansprüche 1 bis 10 zur Herstellung eines Arzneimittels.

12. Verwendung des Chinolinderivats nach einem der Ansprüche 1 bis 10 zur Herstellung eines Arzneimittels zur Behandlung von Erkrankungen, in denen die Angiogenese, Lymphangiogenese oder Vaskulogenese eine Rolle spielen, von Erkrankungen der Blutgefäße, von Erkrankungen, die durch eine Hyperproliferation von Körperzellen hervorgerufen werden, sowie von chronischen oder akuten neurodegenerativen Erkrankungen.

13. Chinolinderivate nach einem der Ansprüche 1 bis 10 für diagnostische Zwecke *in vitro* oder *in vivo* zur Identifizierung von Rezeptren in Geweben mittels Autoradiographie oder PET.

14. Chinolinderivate nach einem der Ansprüche 1 bis 10 zur Verwendung als Inhibitor der Eph-Rezeptor Kinasen.

15. Chinolinderivate nach einem der Ansprüche 1 bis 10 in Form eines pharmazeutischen Präparates zur Verwendung für die enterale, parenterale und orale Applikation.

16. Verfahren zur Herstellung des Chinolinderivats nach einem der Ansprüche 1 -10 mit folgenden Verfahrensschritten gemäß nachstehendem Schema: 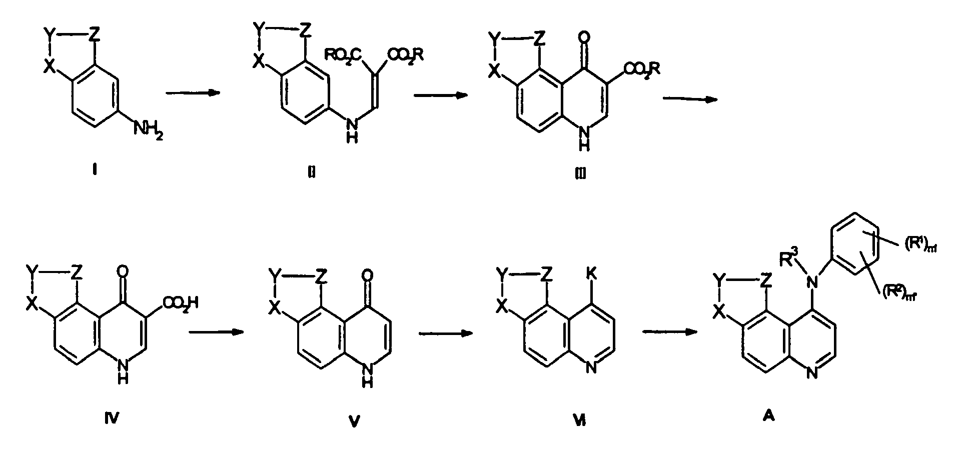 worin
K aus der Gruppe ausgewählt ist, umfassend Halogen und -OS(O)₂CₙF₂ₙ₊₁ mit n = 1 - 3,
R Methyl oder Ethyl ist und
X, Y und Z dieselbe Bedeutung haben wie in der allgemeinen Formel **A**
a) Addieren einer Verbindung mit der allgemeinen Formel **I an** ein Dialkyloxymethylenmalonat unter Bildung einer Verbindung mit der allgemeinen Formel **II,**
b) Zyklisieren der Verbindung mit der allgemeinen Formel **II** zur Verbindung mit der allgemeinen Formel **III**,
c) Verseifen der Verbindung mit der allgemeinen Formel **III** unter Bildung einer Verbindung mit der allgemeinen Formel **IV**,
d) Decarboxylieren der Verbindung mit der allgemeinen Formel **IV** unter Bildung einer Verbindung mit der allgemeinen Formel **V**,
e) Umsetzen der Verbindung mit allgemeinen Formel **V** mit Thionylchlorid oder einem Perfluorsulfonsäureanhydrid unter Bildung einer Verbindung mit der allgemeinen Formel **VI,**
f) Addieren eines Amins mit der allgemeinen Formel (R¹)_{m'},(R2)_{m"}ArNR³H, worin R¹, R², R³, m' und m" dieselben Bedeutungen wie in der allgemeinen Formel **A** haben, an die Verbindung mit der allgemeinen Formel **VI** unter Bildung des Chinolinderivats mit der allgemeinen Formel **A**.

17. Arzneimittel, die mindestens ein Chinolinderivat nach einem der Ansprüche 1 bis 10 sowie geeignete Formulierungs- und Trägerstoffe enthalten.

## Claims

1. Quinoline derivative with general formula A: 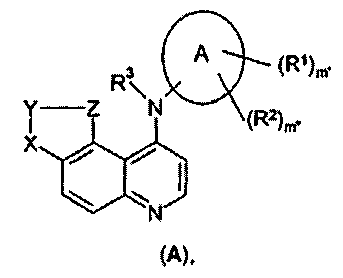 whereby
A is selected from the group that comprises -C₆-C₁₂- aryl, -C₅-C₁₈-heteroaryl, -C₃-C₁₂-cycloalkyl and -C₃-C₁₂-heterocycloalkyl,
R¹ and R² are the same or different and are selected in one or more places, independently of one another, from the group that comprises hydrogen, hydroxy, halogen, nitro, cyano, -C₁-C₆-alkyl, -C₁-C₄-hydroxyalkyl, -C₂-C₆- alkenyl, -C₂-C₆-alkinyl, -C₃-C₁₀-cycloalkyl, -C₃- C₁₂-heterocycloalkyl, -C₆-C₁₂-aryl, -C₅-C₁₈-heteroaryl, -C₁-C₆-alkoxy, -C₁- C₆-alkoxy-C₁-C₆-alkoxy, -C₁-C₆-alkoxy-C₁-C₆-alkyl, - C₁-C₆-alkoxy-C₁-C₆-alkoxy-C₁-C₆-alkyl, -(CH₂)ₙ-C₆- C₁₂-aryl, -(CH₂)n-C₅-C₁₈-heteroaryl, -(CH₂)ₙ-C₃-C₁₀- cycloalkyl, - (CH₂)ₙ-C₃-C₁₂-heterocycloalkyl, - phenylene- (CH₂)ₚ-R⁶, -(CH₂)ₚPO₃(R⁶)₂, -(CH₂)ₚ-NR⁵R⁶, -(CH₂)ₚ-NR⁴COR⁵, - (CH₂)ₚ-NR⁴CSR⁵, -(CH₂)ₚ-NR⁴S(O)R⁵, -(CH₂)ₚ-NR⁴S(O)₂R⁵, -(CH²)ₚ- NR⁴CONR⁵R⁶, -(CH₂)ₚ-NR⁴COOR⁵, - (CH₂)ₚ-NR⁴C(NH)NR⁵R⁶, - (CH₂)ₚ- NR⁴CSNR⁵R⁶, -(CH₂)ₚ-NR⁴S(O)NR⁵R⁶, -(CH₂)ₚ-NR⁴S(O)₂NR⁵R⁶, -(CH₂)ₚ- COR⁵, -(CH₂)ₚ-CSR⁵, -(CH₂)ₚ-S(O)R⁵, -(CH₂)ₚ-S(O)(NH)R⁵, - (CH₂)ₚ-S(O)₂R⁵, -(CH₂)ₚ-S(O)₂NR⁵R⁶, -(CH₂)ₚ-SO₂OR⁵, -(CH₂)ₚ-CO₂R⁵, - (CH₂)ₚ-CONR⁵R⁶, -(CH₂)ₚ-CSNR⁵R⁶, -OR⁵, -(CH₂)ₚ-SR⁵ and -CR⁵(OH)-R⁶, whereby -C₁-C₆-alkyl, -C₂-C₆-alkenyl, - C₂-C₆-alkinyl, -C₃-C₁₀-cycloalkyl, -C₃-C₁₂- heterocycloalkyl, -C₆-C₁₂-aryl, -C₅-C₁₈-heteroaryl and/or -C₁-C₆-alkoxy are unsubstituted or are substituted in one or more places, independently of one another, with hydroxy, halogen, nitro, cyano, phenyl, -NR⁵R⁶, alkyl and/or -OR⁵, whereby the carbon skeleton of the -C₃-C₁₀-cycloalkyl and the -C₁-C₁₀-alkyl can contain nitrogen, oxygen or sulfur atoms and/or C=O groups and/or one or more double bonds in one or more places, independently of one another, and/or R¹ and R² optionally form a bridge with one another that consists of 3-10 methylene units, whereby up to two methylene units are optionally replaced by 0, S and/or -NR⁴,
X, Y, Z are the same or different and are selected independently of one another from the group that comprises -CR³= -CR³R⁴-, - C(O)-, -N=, -S-, -O-, -NR³-, -S(O)₂-, -S(O)- and -S(O)NH- and single or double bonds are found between X, Y and Z,
R³ is hydrogen, -C₁-C₁₀-alkyl or -C₁-C₁₀-alkanoyl,
R⁴ is hydrogen or -C₁-C₁₀-alkyl,
R⁵ and R⁶ are the same or different and are selected, independently of one another, from the group that comprises hydrogen, - C₁-C₁₀-alkyl, -C₂-C₁₀-alkenyl, -C₁-C₁₀-alkinyl, -C₁- C₆-alkoxy, -C₃-C₁₀-cycloalkyl, -C₃-C₁₂- heterocycloalkyl, -C₆-C₁₂-aryl and -C₅-C₁₈- heteroaryl, whereby -C₁-C₁₀-alkyl, -C₂-C₁₀-alkenyl, -C₂-C₁₀-alkinyl, -C₁-C₆-alkoxy, -C₃-C₁₀-cycloalkyl, -C₃-C₁₂-heterocycloalkyl, -C₆-C₁₂-aryl and/or -C₅- C₁₈-heteroaryl are unsubstituted or are substituted in one or more places, independently of one another, with hydroxy, halogen, cyano, nitro, -OR⁷, -NR⁷R⁸, -C(O)NR⁷R⁸, -C(O)OR⁷ and/or -C₁-C₆-alkyl, whereby - C₁-C₆-alkyl is unsubstituted or is substituted in one or more places, independently of one another, with halogen, hydroxy, cyano, -NR⁷R⁸, -OR⁷ and/or phenyl; and/or R⁵ and R⁶ optionally form a bridge with one another that consists of 3-10 methylene units, whereby up to two methylene units optionally are replaced with O, S and/or NR⁴,
R⁷ and R⁸ are the same or different and are selected, independently of one another, from the group that comprises hydrogen, -C₁-C₄-alkyl, -C₆-C₁₂-aryl and -C₅-C₁₈-heteroaryl, whereby alkyl, aryl, or heteroaryl is unsubstituted or is substituted in one or more places, independently of one another, with halogen and/or alkoxy, or R⁷ and R⁸ optionally form a bridge with one another that consists of 3-10 methylene units, whereby up to two methylene units optionally are replaced with O, S and/or -NR⁴;
m', m" = 0-4, independently of one another,
n = 1-6,
p = 0-6, as well as
their N-oxides, solvates, hydrates, stereoisomers, diastereomers, enantiomers and salts, provided that if X, Y, and Z, independently of one another, mean one, two or three N,
1. The skeleton in partial grouping X-Y-Z is not N-CH-N, CH-N-N or N-N-N, and
2. X is not NH, if Y and Z are simultaneously CH in each case.

2. Quinoline derivative according to Claim 1, **characterized in that** if X, Y, and Z, independently of one another, mean one, two or three N,
1. The skeleton in partial grouping X-Y-Z is not N-N-CH, N-CH-N, CH-N-N or N-N-N, and
2. X is not NH, if Y and Z are simultaneously CH in each case.

3. Quinoline derivative according to Claim 1 and/or 2, **characterized in that** A is phenyl.

4. Quinoline derivative according to Claim 3, **characterized in that**
R¹ and R² are the same or different and are selected in one or more places, independently of one another, from the group that comprises hydrogen, hydroxy, halogen, nitro, cyano, -C₁-C₆-alkyl, -C₁-C₄- hydroxyalkyl, -C₆-C₁₂-aryl, -C₁-C₆-alkoxy, -NR⁵R⁶, - NR⁴COR⁵, -NR⁴S(O)R⁵, -NR⁴S(O)₂R⁵, -NR⁴CONR⁵R⁶, - NR⁴S(O)NR⁵R⁶, -NR⁴S(O)₂NR⁵R⁶, -COR⁵, COOR⁵, -S(O)R⁵, -S(O)(NH)R⁵, -S(O)₂R⁵, -S(O)₂NR⁵R⁶, -CO₂R⁵, -CONR⁵R⁶, -OR⁵ and -CR⁵(OH)-R⁶, and
m', m" = 0-3, independently of one another.

5. Quinoline derivative according to one of the preceding claims, **characterized in that** X, Y and Z, independently of one another, are selected from the group that comprises - CR⁴=, -CR⁴R⁵, -C(O)-, -N=, -S-, -0-, -NR⁴-, -S(O)₂-, -S(O)- and - S(O)NH-, whereby N, S or 0 do not occur in several places in the ring.

6. Quinoline derivative according to one of the preceding claims, **characterized in that** X, Y and Z stand for -S(O)₂-, -S-, -NH-, -CH=, -C(CH₃)=, and/or -CH₂-.

7. Quinoline derivative according to one of the preceding claims, **characterized in that** the skeleton in partial grouping X-Y-Z is selected from the group that comprises -S-CH=CH-, -S-C(C₁-C₆-alkyl)=N-, -S(O)₂-CH₂-CH₂- and -CH=CH-S-.

8. Quinoline derivative according to one of the preceding claims, **characterized in that** R³ is hydrogen.

9. Quinoline derivative according to one of the preceding claims, **characterized in that**
A is phenyl,
R¹ and R² are the same or different and are selected in
one or more places, independently of one another, from the group that comprises hydrogen, hydroxy, halogen, nitro, amino, cyano, -C₁-C₆-alkyl, -C₁-C₄-hydroxyalkyl, - C₁-C₆-alkoxy, -C₁-C₄-alkyl-CO-NH-, -NH-C(O)-NH- aryl, -COOR⁵, -CR⁵(OH)-R⁶ and -CONH₂, and
m', m" are 0-3, independently of one another.

10. Quinoline derivative according to Claim 9, **characterized in that**
R¹ and R² are the same or different and are selected in
one or more places, independently of one another, from the group that comprises hydrogen, hydroxy, halogen, nitro, amino, cyano, -CH₃, -C₂H₅, CH₃O-, C₂H₅O-, HOCH₂-, CH₃CONH-, -NH-C(O)-NH-phenyl, -COOH and -CONH₂.

11. Use of the quinoline derivative according to one of Claims 1 to 10 for the production of a pharmaceutical agent.

12. Use of the quinoline derivative according to one of Claims 1 to 10 for the production of a pharmaceutical agent for treating diseases in which angiogenesis, lymphangiogenesis or vasculogenesis plays a role, for treating diseases of the blood vessels, for treating diseases that are caused by a hyperproliferation of body cells, as well as for treating chronic or acute neurodegenerative diseases.

13. Quinoline derivatives according to one of Claims 1 to 10 for diagnostic purposes *in vitro* or *in vivo* for identifying receptors in tissues by means of autoradiography or PET.

14. Quinoline derivatives according to one of Claims 1 to 10 for use as an inhibitor of the Eph-receptor kinases.

15. Quinoline derivatives according to one of Claims 1 to 10 in the form of a pharmaceutical preparation for use for enteral, parenteral and oral administration.

16. Process for the production of the quinoline derivative according to one of claims 1 to 10 with the following process steps according to the diagram below: 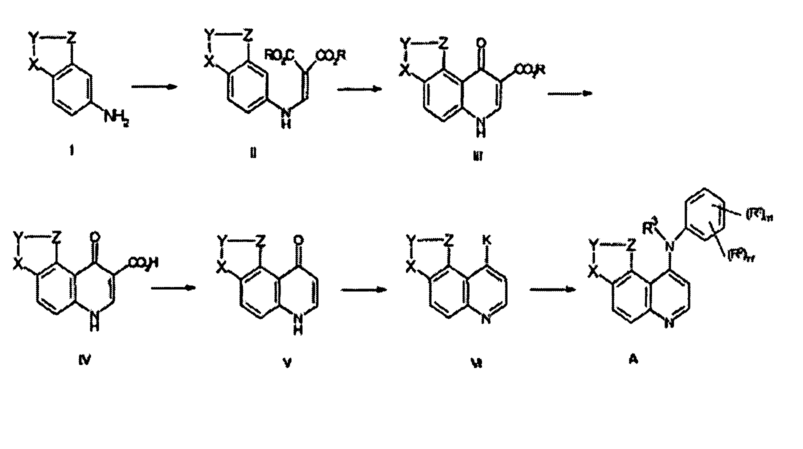 in which
K is selected from the group that comprises halogen and -OS(O)₂CₙF₂ₙ₊₁
with n = 1-3,
R is methyl or ethyl, and
X, Y and Z have the same meaning as in general formula **A**
a) Addition of a compound with general formula **I** to a dialkyloxymethylene malonate with the formation of a compound with general formula **II,**
b) Cyclization of the compound with general formula **II** to the compound with general formula **III,**
c) Saponification of the compound with general formula **III** with the formation of a compound with general formula **IV,**
d) Decarboxylation of the compound with general formula **IV** with the formation of a compound with general formula **V,**
e) Reaction of the compound with general formula **V** with thionyl chloride or a perfluorosulfonic acid anhydride with the formation of a compound with general formula **VI,**
f) Addition of an amine with general formula (R¹)_{m'}, (R²)_{m"}ArNR³H, in which R¹, R², R³, m' and m" have the same meanings as in general formula **A,** to the compound with general formula **VI** with the formation of the quinoline derivative with general formula **A.**

17. Pharmaceutical agents that contain at least one quinoline derivative according to one of Claims 1 to 10 as well as suitable formulation substances and vehicles.

## Revendications

1. Dérivé de quinoléine de formule générale A : 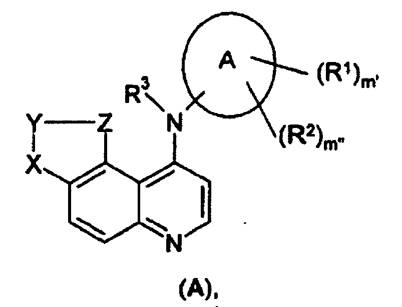 dans laquelle
A est choisi dans l'ensemble comprenant les groupes aryle en C₆-C₁₂, hétéroaryle en C₅-C₁₈, cycloalkyle en C₃-C₁₂ et hétérocycloalkyle en C₃-C₁₂,
R¹ et R² sont identiques ou différents et choisis, une ou plusieurs fois indépendamment l'un de l'autre, dans l'ensemble comprenant un atome d'hydrogène ou d'halogène, les groupes hydroxy, nitro, cyano, alkyle en C₁-C₆, hydroxyalkyle en C₁-C₄, alcényle en C₂-C₆, alcynyle en C₂-C₆, cycloalkyle en C₃-C₁₀, hétérocycloalkyle en C₃-C₁₂, aryle en C₆-C₁₂, hétéroaryle en C₅-C₁₈, alcoxy en C₁-C₆, alcoxy(C₁-C₆)-alcoxy(C₁-C₆), alcoxy(C₁-C₆)- alkyle(C₁-C₆), alcoxy(C₁-C₆)-alcoxy-(C₁-C₆)- alkyle(C₁-C₆), -(CH₂)ₙ-aryle(C₆-C₁₂), -(CH₂)ₙ- hétéroaryle(C₅-C₁₈₎, -(CH₂)ₙ₋cycloalkyle- (C₃-C₁₀), -(CH₂)ₙ-hétérocycloalkyle(C₃-C₁₂), -phénylène-(CH₂)ₚ-R⁶, -(CH₂)ₚPO₃(R⁶)₂, -(CH₂)ₚ-NR⁵R⁶, - (CH₂)ₚ-NR⁴COR⁵, -(CH₂)ₚ-NR⁴CSR⁵, -(CH₂)ₚ-NR⁴S(O)R⁵, -(CH₂)ₚ-NR⁴S(O)₂R⁵, -(CH₂)ₚ-NR⁴CONR⁵R⁶, -(CH₂)ₚ-NR⁴COOR⁵, -(CH₂)ₚ-NR⁴C(NH)NR⁵R⁶, -(CH₂)ₚ-NR⁴CSNR⁵R⁶, -(CH₂)ₚ-NR⁴S(O)NR⁵R⁶, -(CH₂)ₚ-NR⁴S(O)₂NR⁵R⁶, -(CH₂)ₚ-COR⁵, -(CH₂)ₚ-CSR⁵, -(CH₂)ₚ-S(O)R⁵, -(CH₂)ₚ-S(O)(NH)R⁵, -(CH₂)ₚ-S(O)₂R⁵, -(CH₂)ₚ-S(O)₂NR⁵R⁶, -(CH₂)ₚ-SO₂OR⁵, -(CH₂)ₚ-CO₂R⁵, -(CH₂)ₚ-CONR⁵R⁶, -(CH₂)ₚ-CSNR⁵R⁶, -OR⁵, -(CH₂)ₚ-SR⁵ et -CR⁵(OH)-R⁶, les groupes alkyle en C₁-C₆, alcényle en C₂-C₆, alcynyle en C₂-C₆, cycloalkyle en C₃-C₁₀, hétérocycloalkyle en C₃-C₁₂, aryle en C₆-C₁₂, hétéroaryle en C₅-C₁₈ et/ou alcoxy en C₁-C₆ étant non substitués ou une ou plusieurs fois substitués, indépendamment les uns des autres, par hydroxy, halogéno, nitro, cyano, phényle, -NR⁵R⁶, alkyle et/ou -OR⁵, le squelette carboné du groupe cycloalkyle en C₃-C₁₀ et du groupe alkyle en C₁-C₁₀ pouvant comporter un ou plusieurs atomes d'azote, d'oxygène ou de soufre et/ou groupes C=O, indépendamment les uns des autres, et/ou une ou plusieurs doubles liaisons, et/ou R¹ et R² formant éventuellement l'un avec l'autre un pont de 3-10 groupes méthylène, jusqu'à deux groupes méthylène étant éventuellement remplacés par 0, S et/ou -NR⁴,
X, Y, Z sont identiques ou différents et choisis, chacun indépendamment, dans l'ensemble comprenant -CR³=, -CR³R⁴-, -C(O)-, -N=, -S-, -O-, -NR³-, -S(O)₂-, -S(O)- et -S(O)NH-, et des liaisons simples ou doubles se trouvent entre X, Y et Z,
R³ est un atome d'hydrogène, un groupe alkyle en C₁-C₁₀ ou alcanoyle en C₁-C₁₀,
R⁴ est un atome d'hydrogène ou un groupe alkyle en C₁-C₁₀,
R⁵ et R⁶ sont identiques ou différents et choisis, indépendamment l'un de l'autre, dans l'ensemble comprenant un atome d'hydrogène, les groupes alkyle en C₁-C₁₀, alcényle en C₂-C₁₀, alcynyle en C₂-C₁₀, alcoxy en C₁-C₆, cycloalkyle en C₃-C₁₀, hétérocycloalkyle en C₃-C₁₂, aryle en C₆-C₁₂ et hétéroaryle en C₅-C₁₈, les groupes alkyle en C₁-C₁₀, alcényle en C₂-C₁₀, alcynyle en C₂-C₁₀, alcoxy en C₁-C₆, cycloalkyle en C₃-C₁₀, hétérocycloalkyle en C₃-C₁₂, aryle en C₆-C₁₂ et/ou hétéroaryle en C₅-C₁₈ étant non substitués ou une ou plusieurs fois substitués, indépendamment les uns des autres, par hydroxy, halogéno, cyano, nitro, -OR⁷, -NR⁷R⁸, -C(O)NR⁷R⁸, -C(O)OR⁷ et/ou alkyle en C₁-C₆, le groupe alkyle en C₁-C₆ étant non substitué ou une ou plusieurs fois substitué, indépendamment les uns des autres, par halogéno, hydroxy, cyano, -NR⁷R⁸, -OR⁷ et/ou phényle ; et/ou R⁵ et R⁶ éventuellement forment l'un avec l'autre un pont de 3- 10 groupes méthylène, jusqu'à deux groupes méthylène étant éventuellement remplacés par O, S et/ou NR⁴ ;
R⁷, R⁸ sont identiques ou différents et choisis, indépendamment l'un de l'autre, dans l'ensemble comprenant un atome d'hydrogène, les groupes alkyle en C₁-C₄, aryle en C₆-C₁₂ et hétéroaryle en C₅-C₁₈, le groupe alkyle, aryle, hétéroaryle étant non substitué ou une ou plusieurs fois substitué, chacun indépendamment, par un atome d'halogène et/ou par un groupe alcoxy, ou R⁷ et R⁸ éventuellement forment l'un avec l'autre un pont de 3-10 groupes méthylène, jusqu'à deux groupes méthylène étant éventuellement remplacés par O, S et/ou -NR⁴,
m', m" = indépendamment l'un de l'autre, 0 - 4,
n =1 - 6,
p = 0 - 6, ainsi que
leurs N-oxydes, produits de solvatation, hydrates, stéréoisomères, diastéréoisomères, énantiomères et sels,
étant entendu que lorsque X, Y, Z représentent, chacun indépendamment, un, deux ou trois atomes d'azote,
1. le squelette dans le groupement partiel X-Y-Z n'est pas N-CH-N, CH-N-N ni N-N-N et
2. X n'est pas NH lorsque Y et Z représentent simultanément CH.

2. Dérivé de quinoléine selon la revendication 1, **caractérisé en ce que** lorsque X, Y, Z représentent, chacun indépendamment, un, deux ou trois atomes d'azote,
1. le squelette dans le groupement partiel X-Y-Z n'est pas N-N-CH, N-CH-N, CH-N-N ni N-N-N et
2. X n'est pas NH lorsque Y et Z représentent simultanément CH.

3. Dérivé de quinoléine selon la revendication 1 et/ou la revendication 2, **caractérisé en ce que** A est le groupe phényle.

4. Dérivé de quinoléine selon la revendication 3, **caractérisé en ce que**
R¹ et R² sont identiques ou différents et choisis, une ou plusieurs fois indépendamment l'un de l'autre, dans l'ensemble comprenant un atome d'hydrogène ou d'halogène, les groupes hydroxy, nitro, cyano, alkyle en C₁-C₆, hydroxyalkyle en C₁-C₄, aryle en C₆-C₁₂, alcoxy en C₁-C₆, -NR⁵R⁶, NR⁴COR⁵, -NR⁴S(O)R⁵, -NR⁴S(O)₂R⁵, -NR⁴CONR⁵R⁶, -NR⁴S(O)NR⁵R⁶, -NR⁴S(O)₂NR⁵R⁶, -COR⁵, COOR⁵, -S(O)R⁵, -S(O)(NH)R⁵, -S(O)₂R⁵_{,} -S(O)₂NR⁵R⁶, -CO₂R⁵, -CONR⁵R⁶, -OR⁵ et -CR⁵(OH)R⁶, et
m', m" = indépendamment l'un de l'autre 0 - 3.

5. Dérivé de quinoléine selon l'une quelconque des revendications précédentes, **caractérisé en ce que** X, Y et Z sont choisis, chacun indépendamment, dans l'ensemble comprenant -CR⁴=, -CR⁴R⁵-, -C(O)-, -N=, -S-, -O-, -NR⁴-, -S(O)₂-, -S(O)- et -S(O)NH-, N, S ou O n'apparaissant pas plusieurs fois dans le cycle.

6. Dérivé de quinoléine selon l'une quelconque des revendications précédentes, **caractérisé en ce que** X, Y et Z représentent -S(O)₂-, -S-, -NH-, -CH=, -C(CH₃)= et/ou -CH₂-.

7. Dérivé de quinoléine selon l'une quelconque des revendications précédentes, **caractérisé en ce que** le squelette dans le groupement partiel X-Y-Z est choisi dans l'ensemble comprenant -S-CH=CH-, -S-C[alkyle(C₁-C₆)]=N-, -S(O)₂-CH₂-CH₂- et -CH=CH-S-.

8. Dérivé de quinoléine selon l'une quelconque des revendications précédentes, **caractérisé en ce que** R³ est un atome d'hydrogène.

9. Dérivé de quinoléine selon l'une quelconque des revendications précédentes, **caractérisé en ce que**
A représente le groupe phényle,
R¹ et R² sont identiques ou différents et choisis, une ou plusieurs fois indépendamment l'un de l'autre, dans l'ensemble comprenant un atome d'hydrogène ou d'halogène, un groupe hydroxy, nitro, amino, cyano, alkyle en C₁-C₆, hydroxyalkyle en C₁-C₄, alcoxy en C₁-C₆, alkyl(C₁-C₄)-CO-NH-, -NH-C(O)-NH-aryle, -COOR⁵, -CR⁵(OH)-R⁶ et -CONH₂, et
m', m" représentent, indépendamment l'un de l'autre, 0 - 3.

10. Dérivé de quinoléine selon la revendication 9, **caractérisé en ce que**
R¹ et R² sont identiques ou différents et choisis, une ou plusieurs fois indépendamment l'un de l'autre, dans l'ensemble comprenant un atome d'hydrogène ou d'halogène, un groupe hydroxy, nitro, amino, cyano, -CH₃, -C₂H₅, CH₃O-, C₂H₅O-, HOCH₂-, CH₃CONH-, -NH-C(O)-NH- phényle, -COOH et -CONH₂.

11. Utilisation du dérivé de quinoléine selon l'une quelconque des revendications 1 à 10, pour la fabrication d'un médicament.

12. Utilisation du dérivé de quinoléine selon l'une quelconque des revendications 1 à 10, pour la fabrication d'un médicament destiné au traitement de maladies dans l'angiogenèse, la lymphangiogenèse ou la vasculogenèse jouent un rôle, de maladies des vaisseaux sanguins, de maladies qui sont provoquées par une hyperprolifération de cellules corporelles, ainsi que de maladies neurodégénératives chroniques ou aiguës.

13. Dérivés de quinoléine selon l'une quelconque des revendications 1 à 10, à des fins de diagnostic in vitro ou in vivo pour l'identification de récepteurs dans des tissus par autoradiographie ou PET.

14. Dérivés de quinoléine selon l'une quelconque des revendications 1 à 10 pour utilisation en tant qu'inhibiteur des récepteur Eph kinases.

15. Dérivés de quinoléine selon l'une quelconque des revendications 1 à 10, sous forme d'une préparation pharmaceutique destinée à l'utilisation pour l'administration entérale, parentérale ou orale.

16. Procédé pour la préparation du dérivé de quinoléine selon l'une quelconque des revendications 1 à 10, comprenant les étapes suivantes du procédé selon le schéma ci-après : 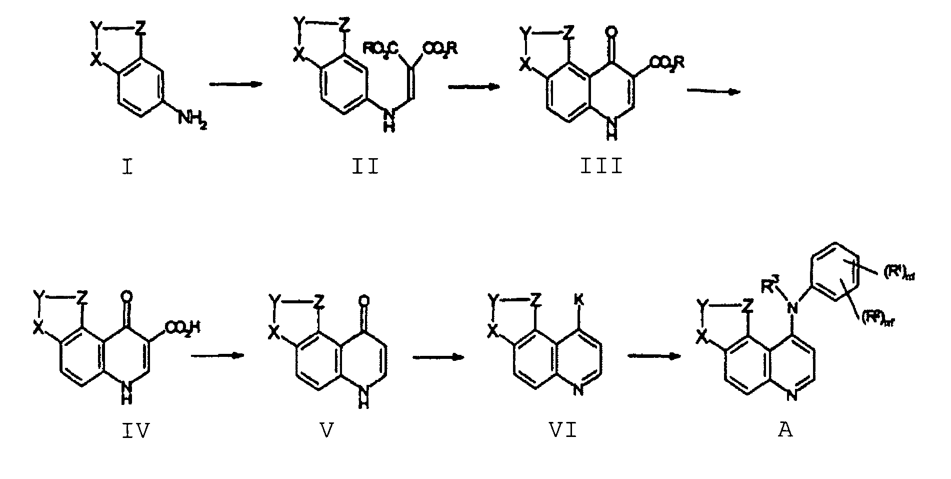 dans lequel
K est choisi dans l'ensemble comprenant un atome d'halogène et -OS(O)₂CₙF₂ₙ₊₁, où n = 1 - 3,
R est le groupe méthyle ou éthyle et
X, Y et Z ont les mêmes significations que dans la formule générale A
a) fixation par addition d'un composé de formule générale I sur un oxyméthylène-malonate de dialkyle, avec formation d'un composé de formule générale II,
b) cyclisation du composé de formule générale II pour l'obtention du composé de formule générale III,
c) saponification du composé de formule générale III, avec formation d'un composé de formule générale IV,
d) décarboxylation du composé de formule générale IV, avec formation d'un composé de formule générale V,
e) mise en réaction du composé de formule générale V avec du chlorure de thionyle ou un anhydride perfluorosulfonique, avec formation d'un composé de formule générale VI,
f) fixation par addition d'une amine de formule générale (R¹)_{m'}, (R²)_{m"}-ArNR³H, dans laquelle R¹, R², R³, m' et m" ont les mêmes significations que dans la formule générale A, sur le composé de formule générale VI, avec formation du dérivé de quinoléine de formule générale A.

17. Médicaments, qui contiennent au moins un dérivé de quinoléine selon l'une quelconque des revendications 1 à 10 ainsi que des adjuvants de formulation et véhicules appropriés.
